# EUROPEAN PATENT APPLICATION

(11) **EP 4 793 277 A1**
(43) Date of publication of application: **19.08.2026**
(21) Application number: 24876590.1
(22) Date of filing: 10.10.2024
(51) Int. Cl.: C07D 471/04, A61K 31/437, A61P 35/00

(54) **CARBOXYLIC ACID COMPOUND AND PREPARATION METHOD THEREFOR AND USE THEREOF**

(30) Priority: 12.10.2023 CN 202311317697
(71) Applicant: Nutshell Therapeutics (Shanghai) Co., Ltd., Shanghai 201210 (CN)
(72) Inventor: ZHOU, Guoqiang, Shanghai 201210 (CN); YU, Liting, Shanghai 201210 (CN); XI, Caiming, Shanghai 201210 (CN)
(74) Representative: Hirons, Daniel Stuart
(86) International application number: PCT/CN2024/123931
(87) International publication number: WO 2025/077778

(57) **Abstract**

Disclosed in the present invention are a carboxylic acid compound and a use thereof. The present invention provides a compound as shown in formula (I), and an isotopic label thereof, a solvate thereof, a pharmaceutically acceptable salt thereof, a solvate of the pharmaceutically acceptable salt thereof or a prodrug thereof. The compound can enhance the ability of p53 Y220C to bind to DNA and has good inhibitory activity on cancer cell proliferation.

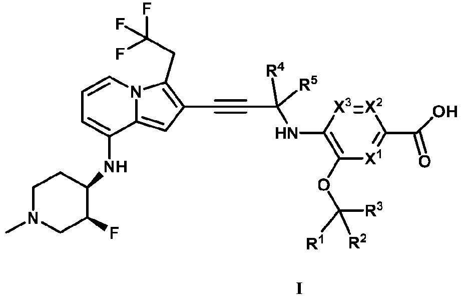

## Description

The present application claims priority to Chinese patent application 2023113176970, filed on October 12, 2023. The contents of the above Chinese patent application are incorporated herein by reference in their entireties.

### TECHNICAL FIELD

The present disclosure belongs to the field of pharmaceuticals, and specifically relates to a class of carboxylic acid compounds, a preparation method therefor, and the use thereof.

### BACKGROUND

The protein product of *TP53* gene (p53) is currently considered as one of the most important tumor suppressor factors. Unlike *RB1*, *CDKN2A*, and *PTEN*, which are lost in tumors through homozygous deletion, *TP53* is often found to have somatic missense mutations (Alexandrova E M et al. "p53 loss-of-heterozygosity is a necessary prerequisite for mutant p53 stabilization and gain-of-function in vivo". Cell Death Dis, 2017, 8(3): e2661-e2661). However, so far, the targeting therapy of p53 mutations has not been validated in clinical treatment (Levine A J. "Targeting therapies for the p53 protein in cancer treatments". Annu Rev Cancer Biol, 2019, 3: 21-34). Cells with *TP53* mutations accumulate high levels of mutant p53 protein, which exerts dominant-negative effects on wild-type (WT) p53 and its homologous proteins p63 and p73, causing them to lose their normal regulatory functions on cell cycle and apoptosis (Boettcher S et al. "A dominant-negative effect drives selection of Tp53 missense mutations in myeloid malignancies". Science, 2019, 365(6453): 599-604). Experimental results of conditional regulation of p53 expression in mouse tissues indicated that restoration of p53 expression could inhibit lymphoma and sarcoma growth through apoptosis in different mouse models (Christophorou M A et al. "Temporal dissection of p53 function in vitro and in vivo"[J]. Nat Genet, 2005, 37(7): 718-726; Martins C.P., Brown-Swigart L., Evan G.I. "Modeling the therapeutic efficacy of p53 restoration in tumors". Cell. 2006; 127: 1323-1334), providing a theoretical basis for leveraging the restoration of p53 normal function for tumor treatment. In addition to enhancing wild-type p53 activity in tumors with intact *Tp53* genotypes, restoring wild-type protein function in tumors expressing mutant p53 is also a promising strategy for cancer treatment (Yu X et al. "Allele-specific p53 mutant reactivation". Cancer cell, 2012, 21(5): 614-625; Chen S et al. "Arsenic trioxide rescues structural p53 mutations through a cryptic allosteric site". Cancer cell, 2021, 39(2): 225-239. e8).

The core domain of wild-type p53 is unstable, with poor thermodynamic and kinetic stability, allowing for rapid transformation between folded and unfolded states. Mutation of p53 core region residues leads to enhanced thermodynamic and kinetic instability, thereby causing the core DNA-binding domain to lose DNA-binding activity. Such effects can be used to design ligands that selectively bind to the native state of p53 protein to reverse the thermodynamic and kinetic denaturation caused by these mutations (Zhang S et al. "Advanced strategies for therapeutic targeting of wild-type and mutant p53 in cancer". Biomolecules. 2022, 12(4): 548). It is worth noting that the special Y220C mutation mediates the formation of a surface pocket away from the core DNA-binding domain (Joerger A C et al. "Structural basis for understanding oncogenic p53 mutations and designing rescue drugs". Proc Natl Acad Sci, 2006, 103(41): 15056-15061), rendering it an ideal target for treating tumors harboring such mutation. So far, many Y220C pocket binding agents have been developed to stabilize the p53 mutant and reactivate its transcriptional activity, but with limited efficacy (Boeckler F M, et al. "Targeted rescue of a destabilized mutant of p53 by an in silico screened drug". Proc Natl Acad Sci, 2008, 105(30): 10360-10365; Guiley K Z et al. "A small molecule reacts with the p53 somatic mutant Y220C to rescue wild-type thermal stability". Cancer Discov, 2022: CD-22). Therefore, for tumor patients with p53 Y220C mutation, there is an urgent need to develop p53 Y220C pocket binding agents with stronger affinity and better druggability.

### SUMMARY

The technical issue to be solved by the present disclosure is the poor druggability of existing p53 Y220C pocket binding agents. Therefore, the present disclosure provides an indolizine carboxylic acid compound and the use thereof. The indolizine carboxylic acid compounds can promote the binding of p53 Y220C with DNA and possess superior inhibitory activity against the proliferation of cancer cells, particularly gastric cancer cells.

The present disclosure provides a compound of formula (I), an isotope-labeled compound thereof, a solvate thereof, a pharmaceutically acceptable salt thereof, a solvate of the pharmaceutically acceptable salt thereof, or a prodrug thereof; 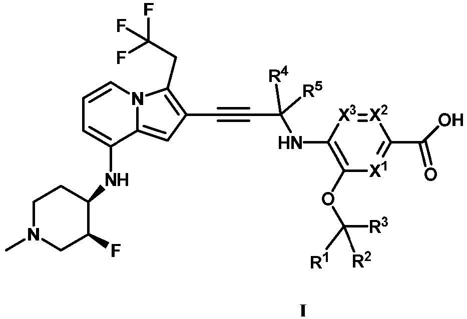 wherein
X¹, X², and X³ are each independently selected from CH and N;
R¹, R², R³, R⁴, and R⁵ are each independently selected from hydrogen and deuterium.

As a preferred technical solution, X¹ is N; X² is CH or N; X³ is CH.

As a preferred technical solution, X¹ is N; X² and X³ are CH.

As a preferred technical solution, R¹, R², R³, R⁴, and R⁵ are each independently selected from hydrogen and deuterium, and at least one of them is deuterium.

As a preferred technical solution, R¹, R², and R³ are deuterium; R⁴ and R⁵ are hydrogen.

As a preferred technical solution, R¹, R², and R³ are hydrogen; R⁴ and R⁵ are deuterium.

As a preferred technical solution, the compound of formula (I) is selected from the following compounds: 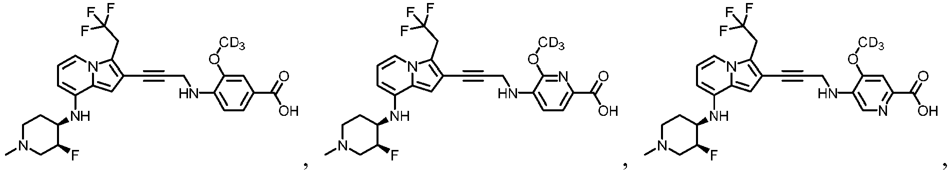 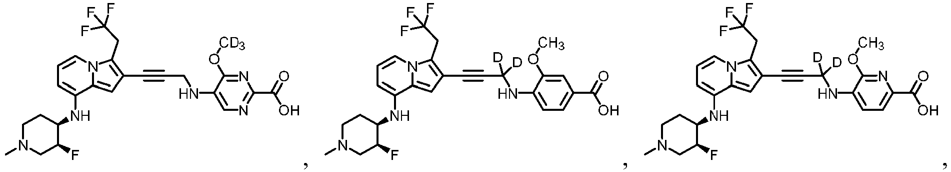 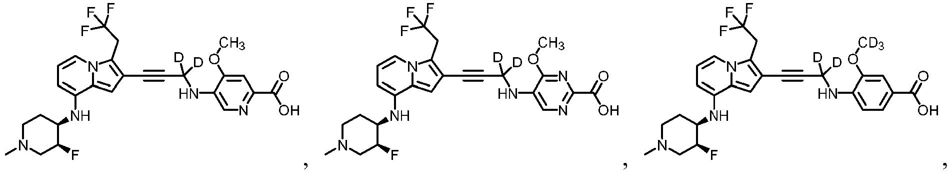 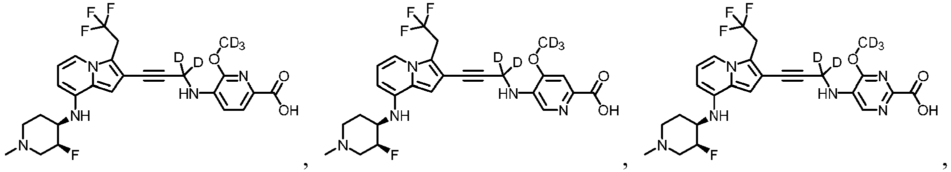 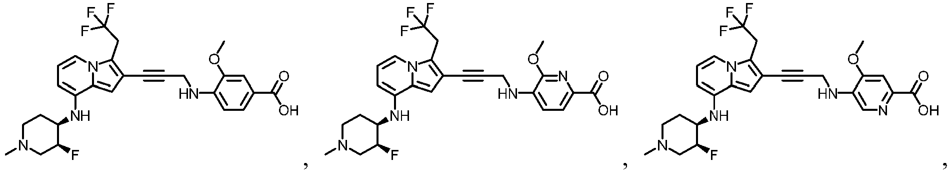 and

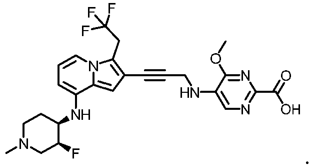

The present disclosure further provides a preparation method for the compound of formula (I), an isotope-labeled compound thereof, an enantiomer thereof, a diastereomer thereof, a solvate thereof, or a pharmaceutically acceptable salt thereof, including the following methods:

### Method 1:

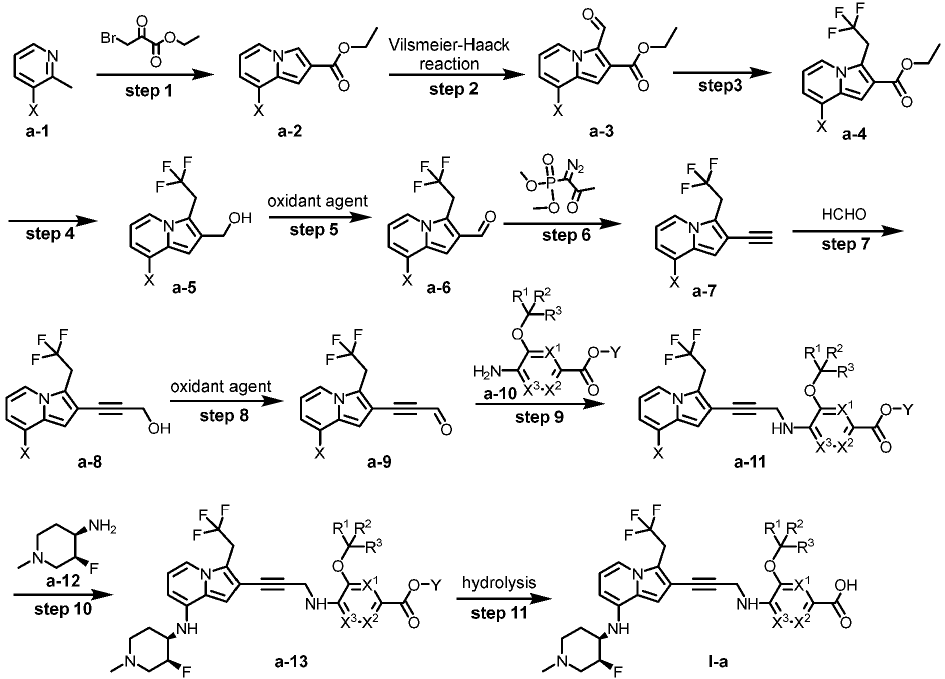

Step 1: Carrying out a cycloaromatization reaction between compound a-1 and ethyl bromopyruvate to obtain compound a-2, wherein X is selected from Cl, Br, and I;
Step 2: Subjecting compound a-2 to a Vilsmeier-Haack reaction to obtain compound a-3;
Step 3: Reacting compound a-3 with a fluorinating reagent to obtain compound a-4, wherein the fluorinating reagent may be methyl fluorosulfonyldifluoroacetate, (triphenylphosphonio)difluoroacetate, or tetrabutylammonium fluoride;
Step 4: Subjecting compound a-4 to a reduction reaction of the ester group in the presence of a reducing agent to obtain compound a-5, wherein the reducing agent may be sodium borohydride, lithium borohydride, lithium aluminum hydride, or diisobutylaluminium hydride;
Step 5: Oxidizing compound a-5 in the presence of an oxidizing agent to obtain compound a-6, wherein the oxidizing agent may be Dess-Martin periodinane, Swern oxidation reagent, pyridinium chlorochromate, manganese dioxide, or sulfur trioxide/pyridine;
Step 6: Reacting compound a-6 with Gilbert reagent to obtain compound a-7;
Step 7: Reacting compound a-7 with formaldehyde under the catalysis of a metal reagent to obtain compound a-8, wherein the metal reagent may be a reagent containing Cu, such as tetrakis(acetonitrile)copper(I) hexafluorophosphate;
Step 8: Oxidizing compound a-8 into aldehyde a-9 in the presence of an oxidizing agent, wherein the oxidizing agent may be Dess-Martin periodinane, Swern oxidation reagent, pyridinium chlorochromate, manganese dioxide, or sulfur trioxide/pyridine;
Step 9: Carrying out a reductive amination reaction between compound a-9 and compound a-10 to obtain compound a-11, wherein Y is selected from C₁-C₄ alkyl; R¹, R², R³, X¹, X², and X³ are defined as above;
Step 10: Carrying out a metal reagent-catalyzed coupling reaction between compound a-11 and amine a-12 to obtain compound a-13, wherein the metal reagent may be a reagent containing Pd or Cu, such as Brettphos Pd G3, Brettphos Pd G4, or cuprous iodide;
Step 11: Subjecting compound a-13 to a hydrolysis reaction under acidic or basic conditions to obtain (I-a), wherein the acidic conditions may be achieved in the presence of inorganic acids (*e.g.*, HCl, HBr, or H₂SO₄) or organic acids (*e.g.*, trifluoroacetic acid), and the basic conditions may be achieved in the presence of inorganic bases (*e.g.*, LiOH, NaOH, or KOH);

### Method 2:

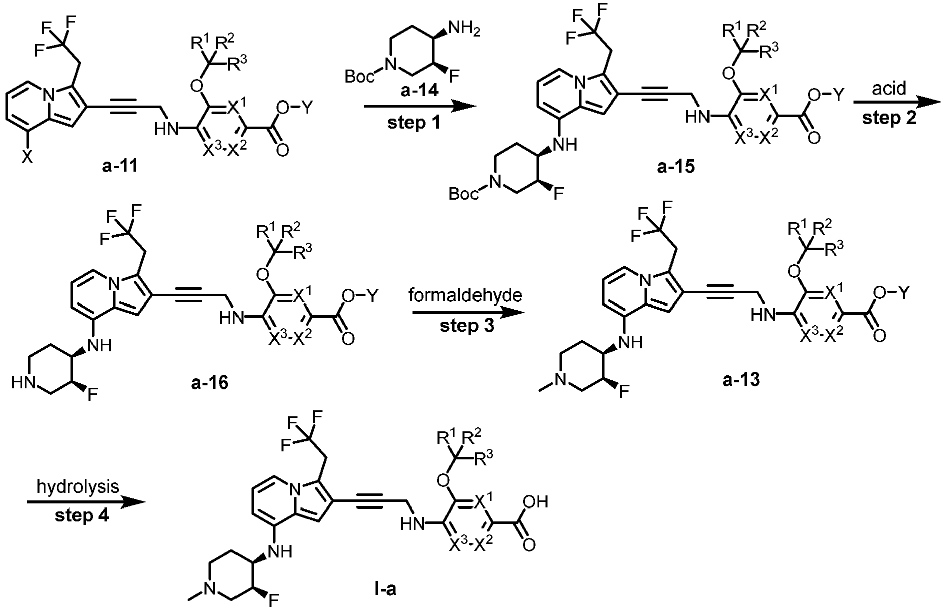

Step 1: Carrying out a metal reagent-catalyzed coupling reaction between compound a-11 and amine a-14 to obtain compound a-15, wherein X is selected from Cl, Br, and I; Y is selected from C₁-C₄ alkyl; R¹, R², R³, X¹, X², and X³ are defined as above; The metal reagent may be a reagent containing Pd or Cu, such as Brettphos Pd G3, Brettphos Pd G4, or cuprous iodide;
Step 2: Removing Boc protecting group from compound a-15 under acidic conditions to obtain compound a-16, wherein the acidic conditions may be achieved in the presence of inorganic acids (*e.g.*, HCl, HBr, or H₂SO₄), organic acids (*e.g.*, trifluoroacetic acid), Lewis acids (*e.g.*, zinc bromide), or other reagents (*e.g.*, iodotrimethylsilane);
Step 3: Carrying out a reductive amination reaction between compound a-16 and formaldehyde to obtain compound a-13;
Step 4: Subjecting compound a-13 to a hydrolysis reaction under acidic or basic conditions to obtain (I-a), wherein the acidic conditions may be achieved in the presence of inorganic acids (*e.g.*, HCl, HBr, or H₂SO₄) or organic acids (*e.g.*, trifluoroacetic acid), and the basic conditions may be achieved in the presence of inorganic bases (*e.g.*, LiOH, NaOH, or KOH);

### Method 3:

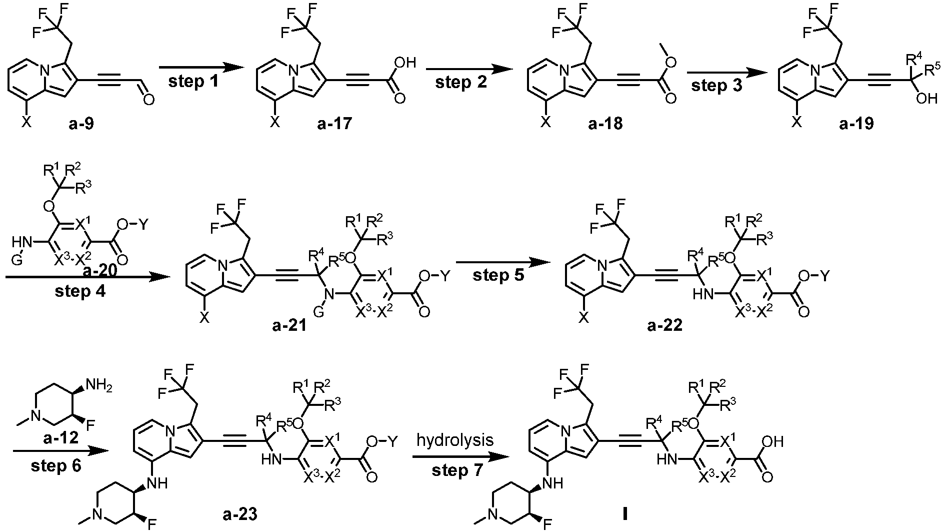

Step 1: Subjecting compound a-9 to Pinnick oxidation to obtain compound a-17;
Step 2: Subjecting compound a-17 to an esterification reaction to obtain compound a-18;
Step 3: Subjecting compound a-18 to a reduction reaction to obtain compound a-19, wherein the reducing agent may be a reagent containing sodium (lithium) borohydride or lithium aluminum hydride, *etc.*, as well as their deuterated reagents; R⁴ and R⁵ are defined as above;
Step 4: Subjecting compound a-19 to a Mitsunobu coupling reaction to obtain compound a-21, wherein Y is selected from C₁-C₄ alkyl; R¹, R², R³, R⁴, R⁵, X¹, X², and X³ are defined as above; G is an electron-withdrawing protecting group, such as *p*-toluenesulfonyl, *p-*nitrobenzenesulfonyl, or *m*-nitrobenzenesulfonyl;
Step 5: Removing the protecting group G from compound a-21 to obtain a-22;
Step 6: Carrying out a metal reagent-catalyzed coupling reaction between compound a-22 and amine a-12 to obtain compound a-23, wherein the metal reagent may be a reagent containing Pd or Cu, such as Brettphos Pd G3, Brettphos Pd G4, or cuprous iodide;
Step 7: Subjecting compound a-23 to a hydrolysis reaction under acidic or basic conditions to obtain (I), wherein the acidic conditions may be achieved in the presence of inorganic acids (*e.g.*, HCl, HBr, or H₂SO₄) or organic acids (*e.g.*, trifluoroacetic acid), and the basic conditions may be achieved in the presence of inorganic bases (*e.g.*, LiOH, NaOH, or KOH).

The present disclosure further provides a pharmaceutical composition comprising the compound of formula (I), an isotope-labeled compound thereof, an enantiomer thereof, a diastereomer thereof, a solvate thereof, or a pharmaceutically acceptable salt thereof, and a pharmaceutical excipient.

The present disclosure further provides the use of the compound of formula (I), an isotope-labeled compound thereof, an enantiomer thereof, a diastereomer thereof, a solvate thereof, or a pharmaceutically acceptable salt thereof, or the pharmaceutical composition in the manufacture of a p53 mutant pocket binding agent or a medicament for the treatment and/or prevention of a disease related to p53 mutants.

In a preferred technical solution, the described use satisfies one or more of the following conditions:
(1) The p53 mutant has a mutation at amino acid 220, such as p53 Y220C;
(2) The pocket binding agent promotes the ability of p53 mutants to bind to DNA;
(3) The disease related to p53 mutants is cancer, such as breast cancer, gastric cancer, lung cancer, or ovarian cancer.

The present disclosure further provides the use of the compound of formula (I), an isotope-labeled compound thereof, an enantiomer thereof, a diastereomer thereof, a solvate thereof, or a pharmaceutically acceptable salt thereof, or the pharmaceutical composition in the manufacture of a medicament for the treatment and/or prevention of cancer.

The cancer is, for example, breast cancer, gastric cancer, lung cancer, or ovarian cancer.

The present disclosure further provides a method for the treatment and/or prevention of cancer, wherein the method comprises administering to a patient a therapeutically effective amount of the compound of formula (I), an isotope-labeled compound thereof, an enantiomer thereof, a diastereomer thereof, a solvate thereof, or a pharmaceutically acceptable salt thereof.

The term "pharmaceutically acceptable" refers to relatively nontoxic, safe, and suitable for patient use.

The term "pharmaceutically acceptable salt" refers to a salt obtained by reacting a compound with a pharmaceutically acceptable acid or base. When a compound contains relatively acidic functional groups, the corresponding base addition salt may be obtained by contacting a compound with a sufficient amount of pharmaceutically acceptable base in an appropriate inert solvent. When a compound contains relatively basic functional groups, the corresponding acid addition salt may be obtained by contacting a compound with a sufficient amount of pharmaceutically acceptable acid in an appropriate inert solvent. For details, see "Handbook of Pharmaceutical Salts: Properties, Selection, and Use" (P. Heinrich Stahl, Camille G. Wermuth, 2011, 2nd Revised Edition).

The term "solvate" refers to a substance formed by combining a compound with a solvent (including, but not limited to: water, methanol, ethanol, *etc.*). Solvates are classified into stoichiometric solvates and non-stoichiometric solvates.

The term "solvate of the pharmaceutically acceptable salt" refers to a substance formed by combining a compound with a pharmaceutically acceptable acid or base and a solvent (including, but not limited to: water, methanol, ethanol, *etc.*). The amount of solvent herein may be stoichiometric or non-stoichiometric.

The "-" at the end of a group indicates that the group is connected to the rest of the molecule through the site. For example, CH₃-C(=O)- refers to an acetyl group.

When the valence bond of a group is marked with a wavy line " ", such as in " 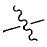 ", the wavy line indicates the point at which the group is connected to the rest of the molecule.

The term "halogen" refers to fluorine, chlorine, bromine, or iodine.

The term "alkyl" refers to a straight or branched, monovalent hydrocarbon group with a specified number of carbon atoms (*e.g.*, C₁-C₆). Alkyl groups include, but are not limited to: methyl, ethyl, *n*-propyl, isopropyl, *n*-butyl, isobutyl, *sec*-butyl, *tert*-butyl, *n*-pentyl, *n*-hexyl, *etc*.

The term "alkoxy" refers to the group R^{X}-O-, where R^{X} is defined as in the term "alkyl". Alkoxy groups include, but are not limited to: methoxy, ethoxy, *n*-propoxy, isopropoxy, *etc*.

The term "therapeutically effective amount" refers to an amount administered to a patient that is sufficient to effectively treat a disease. The therapeutically effective amount will vary depending on the type of compound, type of disease, severity of disease, age of patient, *etc.*, but may be adjusted by those skilled in the art as required.

The term "pharmaceutical excipient" refers to all substances contained in pharmaceutical preparations, except active pharmaceutical ingredients, and is generally divided into two categories: vehicles and additives. For details, see "Pharmacopoeia of the People's Republic of China (2020 Edition)" and "Handbook of Pharmaceutical Excipients" (Paul J Sheskey, Bruno C Hancock, Gary P Moss, David J Goldfarb, 2020, 9th Edition).

The term "treat/treatment" refers to eliminating the cause of a disease or alleviating symptoms.

The term "prevent/prevention" refers to reducing the risk of developing a disease.

The term "patient" refers to any animal, typically a mammal, such as a human, in need of treatment or prevention of a disease. Mammals include, but are not limited to: cattle, horses, sheep, pigs, cats, dogs, mice, rats, rabbits, guinea pigs, monkeys, humans, *etc*.

The term "PG" refers to a protecting group, such as Boc protecting group.

In accordance with common knowledge in the art, the above various preferred conditions may be arbitrarily combined to obtain the various preferred embodiments of the present disclosure.

The reagents and starting materials used in the present disclosure are commercially available.

The positive and progressive effect of the present disclosure is that the compounds of the present disclosure can promote the binding of p53 Y220C with DNA and possess superior inhibitory activity against the proliferation of cancer cells, particularly gastric cancer cells.

### DETAILED DESCRIPTION OF THE PREFERRED EMBODIMENT

The present disclosure is further described through examples below, but is not limited to the scope of the examples provided. In the following examples, experimental methods without specified conditions are selected according to conventional methods and conditions, or according to product instructions.

In each example, ¹HNMR was recorded on a BRUKER AVANCE NEO 400 MHz or JNM-ECZ400s nuclear magnetic resonance spectrometer, with chemical shifts expressed in δ (ppm); Liquid chromatography-mass spectrometry (LCMS) was recorded on a Shimadzu LC-20AD, Agilent 1260, or Agilent 1200 mass spectrometer; Preparative HPLC separation was performed using a WATERS Autop or Shimadzu LC20AR liquid chromatograph.

### Abbreviation

| | | |
|---|---|---|
| BrettPhos Pd G3 | Methanesulfonato(2-dicyclohexylphosphino-3,6-dimethoxy-2',4',6'-tri-i-propyl-1,1'-bipheny)(2'-amino-1,1'-biphenyl-2-yl)palladium(II) | Methanesulfonato(2-dicyclohexylphosphino-3,6-dimethoxy-2',4',6'-tri-i-propyl-1,1'-bipheny)(2'-amino-1,1'-biphenyl-2-yl)palladium(II) |
| BrettPhos Pd G4 | Methanesulfonato(2-dicyclohexylphosphino-3,6-dimethoxy-2',4',6'-tri-i-propyl-1,1'-biphenyl)(2'-methylamino-1,1'-biphenyl-2-yl)palladium(II) | Methanesulfonato(2-dicyclohexylphosphino-3,6-dimethoxy-2',4',6'-tri-i-propyl-1,1'-biphenyl)(2'-methylamino-1,1'-biphenyl-2-yl)palladium(II) |
| DCM | Dichloromethane | Dichloromethane |
| DIBAL-H | Diisobutylaluminium hydride | Diisobutylaluminium hydride |
| DMF | *N,N*-Dimethylformamide | *N,N*-Dimethylformamide |
| DMP | Dess-Martin periodinane | Dess-Martin periodinane |
| DMSO | Dimethyl sulfoxide | Dimethyl sulfoxide |
| EtOAc | Ethyl acetate | Ethyl acetate |
| HATU | 2-(7-Azabenzotriazol-1-yl)-*N,N,N',N'*-tetramethyluronium hexafluorophosphate | 2-(7-Azabenzotriazol-1-yl)-*N,N,N',N'*-tetramethyluronium hexafluorophosphate |
| MTBE | *tert*-Butyl methyl ether | *tert*-Butyl methyl ether |
| MeOH | Methanol | Methanol |
| Pd(dppf)Cl₂ | [1,1'-Bis(diphenylphosphino)ferrocene] dichloropalladium(II) | [1,1'-Bis(diphenylphosphino)ferrocene] dichloropalladium(II) |
| PE | Petroleum ether | Petroleum ether |
| Ruphos | 2-Dicyclohexylphosphino-2',6'-di-iso-propoxy-1,1'-biphenyl | 2-Dicyclohexylphosphino-2',6'-di-iso-propoxy-1,1'-biphenyl |
| SGC | Silica gel column chromatography | Silica gel column chromatography |
| TBAF | Tetrabutylammonium fluoride | Tetrabutylammonium fluoride |
| TEA | Triethylamine | Triethylamine |
| THF | Tetrahydrofuran | Tetrahydrofuran |

### Preparation of intermediates Int-1 and Int-2

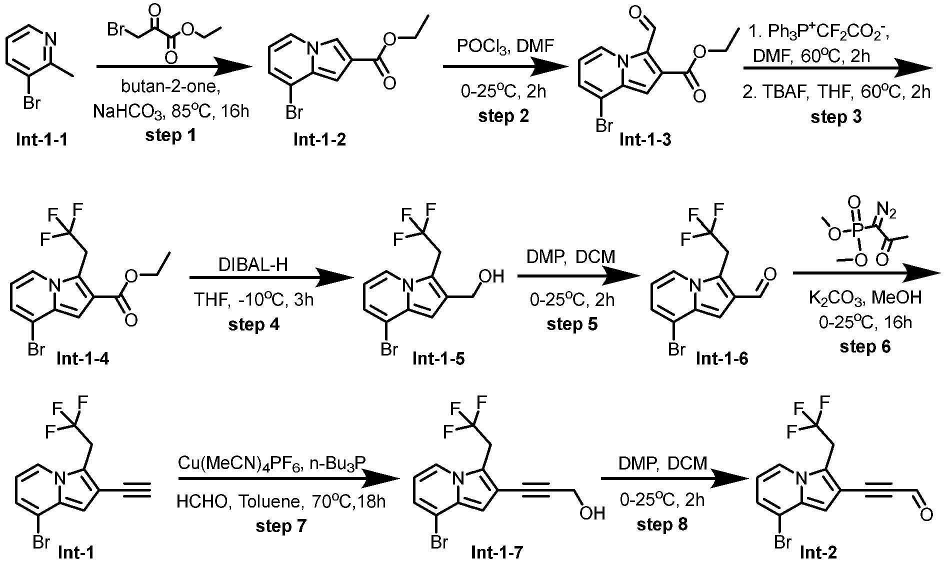

### Preparation of intermediates Int-1 and Int-2

### Step 1: Ethyl 8-bromoindolizine-2-carboxylate

A mixture of 3-bromo-2-methylpyridine **Int-1-1** (500 mg, 2.91 mmol), ethyl 3-bromopyruvate (850 mg, 4.36 mmol), and sodium bicarbonate (561 mg, 6.69 mmol) in butanone (5 mL) was stirred at 85°C for 16 hours, then concentrated to dryness, and purified by SGC (0 to 10% EtOAc in PE) to obtain ethyl 8-bromoindolizine-2-carboxylate **Int-1-2** (190 mg, 24.4% yield) as a gray solid. LCMS calculated for C₁₁H₁₁BrNO₂ [M+H]⁺: m/z = 268.0/270.0; found: 267.9/269.9; ¹H NMR (400 MHz, CDCl₃) *δ* 7.88 (d, *J* = 1.6 Hz, 1H), 7.87-7.83 (m, 1H), 7.03-6.99 (m, 1H), 6.95 (d, *J* = 6.8 Hz, 1H), 6.43 (t, *J* = 7.2 Hz, 1H), 4.37 (q, *J* = 7.2 Hz, 2H), 1.40 (t, *J* = 6.8 Hz, 3H).

### Step 2: Ethyl 8-bromo-3-formylindolizine-2-carboxylate

A solution of ethyl 8-bromoindolizine-2-carboxylate **Int-1-2** (5.34 g, 19.9 mmol) in DCM (130 mL) was added dropwise to a solution of phosphorus oxychloride (5.19 g, 33.8 mmol) in DMF (130 mL) at 0°C. The mixture was stirred at 20°C for 1 hour, then slowly quenched with saturated sodium bicarbonate aqueous solution (300 mL), and extracted with DCM (200 mL). The separated organic layers were combined, dried over Na₂SO₄, filtered, and concentrated to dryness. The residue was purified by SGC (0 to 10% EtOAc in PE) to obtain ethyl 8-bromo-3-formylindolizine-2-carboxylate **Int-1-3** (10.6 g, crude product) as a yellow colloid. LCMS calculated for C₁₂H₁₁BrNO₃ [M+H]⁺: m/z = 296.0/298.0; found: 295.9/297.9.

### Step 3: Ethyl 8-bromo-3-(2,2,2-trifluoroethyl)indolizine-2-carboxylate

A mixture of ethyl 8-bromo-3-formylindolizine-2-carboxylate **Int-1-3** (8.50 g, 15.8 mmol) and 2,2-difluoro-2-triphenylphosphaniumylacetate (11.2 g, 31.5 mmol) in DMF (120 mL) was stirred at 60°C for 2 hours. TBAF (47.3 mL, 47.3 mmol, 1M in THF) was added to the mixture. The reaction mixture was stirred at 60°C for 2 hours, then diluted with water (100 mL), and extracted with MTBE (100 mL × 3). The organic layers were combined, dried over Na₂SO₄, filtered, and concentrated to dryness under reduced pressure. The residue was purified by SGC (0 to 5% EtOAc in PE) to obtain ethyl 8-bromo-3-(2,2,2-trifluoroethyl)indolizine-2-carboxylate **Int-1-4** (2.40 g, 43.4% yield) as a white solid. LCMS calculated for C₁₃H₁₂BrF₃NO₂ [M+H]⁺: m/z = 350.0/352.0; found: 349.9/351.9; ¹H NMR (400 MHz, CDCl₃) *δ* 7.89 (d, *J* = 7.2 Hz, 1H), 7.12 (s, 1H), 7.05 (d, *J* = 6.8 Hz, 1H), 6.56 (t, *J* = 7.2 Hz, 1H), 4.40 (q, *J* = 7.2 Hz, 2H), 4.25 (q, *J* = 10.0 Hz, 2H), 1.42 (t, *J* = 7.2 Hz, 3H).

### Step 4: (8-Bromo-3-(2,2,2-trifluoroethyl)indolizin-2-yl)methanol

DIBAL-H (35.1 mL, 35.1 mmol, 1M in toluene) was added to a mixture of ethyl 8-bromo-3-(2,2,2-trifluoroethyl)indolizine-2-carboxylate **Int-1-4** (4.10 g, 11.7 mmol) in THF (120 mL). The reaction mixture was stirred at -10°C for 3 hours to obtain a yellow solution. The reaction mixture was poured into saturated ammonium chloride aqueous solution (200 mL), then water (200 mL) was added thereto, and the mixture was extracted with EtOAc (150 mL × 3). The organic layers were combined, dried over Na₂SO₄, filtered, and concentrated to dryness under reduced pressure. The residue was purified by SGC (0 to 5% EtOAc in PE) to obtain (8-bromo-3-(2,2,2-trifluoroethyl)indolizin-2-yl)methanol **Int-1-5** (3.55 g, 98.4% yield) as a white solid. LCMS calculated for C₁₁H₁₀BrF₃NO [M+H]⁺: m/z = 308.0/310.0; found: 307.9/309.9; ¹H NMR (400 MHz, CDCl₃) *δ* 7.85 (d, *J* = 7.2 Hz, 1H), 7.00 (d, *J* = 6.8 Hz, 1H), 6.70 (br s, 1H), 6.50 (t, *J* = 7.2 Hz, 1H), 4.84 (s, 2H), 3.83 (q, *J* = 10.4 Hz, 2H).

### Step 5: 8-Bromo-3-(2,2,2-trifluoroethyl)indolizine-2-carbaldehyde

DMP (7.33 g, 17.2 mmol) was added to a solution of (8-bromo-3-(2,2,2-trifluoroethyl)indolizin-2-yl)methanol **Int-1-5** (3.55 g, 11.5 mmol) in DCM (400 mL) at 0°C. The reaction system was warmed to room temperature and stirred for 2 hours. The reaction mixture was poured into saturated sodium bicarbonate aqueous solution (20 mL), then water (200 mL) was added thereto, and the mixture was extracted with DCM (100 mL × 3). The organic layers were combined, dried over Na₂SO₄, filtered, and concentrated to dryness under reduced pressure. The residue was purified by SGC (0 to 7% EtOAc in PE) to obtain 8-bromo-3-(2,2,2-trifluoroethyl)indolizine-2-carbaldehyde **Int-1-6** (2.70 g, 76.5% yield) as a white solid. LCMS calculated for C₁₁H₈BrF₃NO [M+H]⁺: m/z = 306.0/308.0; found: 305.9/307.9.

### Step 6: 8-Bromo-2-ethynyl-3-(2,2,2-trifluoroethyl)indolizine

8-Bromo-3-(2,2,2-trifluoroethyl)indolizine-2-carbaldehyde **Int-1-6** (500 mg, 1.63 mmol) was added to a mixture of dimethyl (1-diazo-2-oxopropyl)phosphonate (470 mg, 2.45 mmol) and potassium carbonate (451 mg, 3.27 mmol) in MeOH (15 mL) at 0°C. The reaction system was warmed to room temperature and stirred for 16 hours. The reaction mixture was diluted with water (50 mL) and extracted with EtOAc (50 mL × 3). The organic layers were combined, dried over Na₂SO₄, filtered, and concentrated to dryness under reduced pressure. The residue was purified by SGC (0 to 3% EtOAc in PE) to obtain 8-bromo-2-ethynyl-3-(2,2,2-trifluoroethyl)indolizine **Int-1** (380 mg, 77.0% yield) as a white solid. LCMS calculated for C₁₂H₈BrF₃N [M+H]⁺: m/z = 302.0/304.0; found: 301.9/303.9; ¹H NMR (400 MHz, CDCl₃) *δ* 7.81 (d, *J* = 7.2 Hz, 1H), 7.03 (d, *J* = 7.2 Hz, 1H), 6.79 (s, 1H), 6.52 (t, *J* = 7.2 Hz, 1H), 3.83 (q, *J* = 10.0 Hz, 2H), 3.25 (s, 1H).

### Step 7: 3-(8-Bromo-3-(2,2,2-trifluoroethyl)indolizin-2-yl)prop-2-yn-1-ol

Under a nitrogen atmosphere, tetrakis(acetonitrile)copper(I) hexafluorophosphate (123 mg, 0.33 mmol) and tributylphosphine (268 mg, 1.32 mmol) were dissolved in toluene (15 mL), and the reaction mixture was stirred at 70°C for 30 minutes. 8-Bromo-2-ethynyl-3-(2,2,2-trifluoroethyl)indolizine **Int-1** (1.00 g, 3.31 mmol) and formaldehyde aqueous solution (0.18 mL, 6.62 mmol, 36% to 38% content) were then added thereto. The mixture was stirred at 70°C overnight. After the completion of the reaction detected by TLC, the mixture was concentrated under vacuum. The residue was purified by SGC (0 to 15% EtOAc in PE) to obtain 3-(8-bromo-3-(2,2,2-trifluoroethyl)indolizin-2-yl)prop-2-yn-1-ol **Int-1-7** (843 mg, 76.7% yield) as a white solid. LCMS calculated for C₁₃H₁₀BrF₃NO [M+H]⁺: m/z = 332.0/334.0; found: 332.3/334.3; ¹H NMR (400 MHz, DMSO-*d*₆) *δ* 8.38 (d, *J* = 7.1 Hz, 1H), 7.19 (d, *J* = 7.1 Hz, 1H), 6.68 (t, *J* = 7.1 Hz, 1H), 6.61 (s, 1H), 5.34 (t, *J* = 6.0 Hz, 1H), 4.34 (d, *J* = 6.0 Hz, 2H), 4.20-4.11 (m, 2H).

### Step 8: 3-(8-Bromo-3-(2,2,2-trifluoroethyl)indolizin-2-yl)propiolaldehyde

DMP (17.4 g, 41.0 mmol) was slowly added portionwise to a solution of 3-(8-bromo-3-(2,2,2-trifluoroethyl)indolizin-2-yl)prop-2-yn-1-ol **Int-1-7** (6.80 g, 20.5 mmol) in DCM (120 mL) at 0°C. The mixture was stirred at room temperature for 2 hours. The reaction mixture was quenched with saturated sodium bicarbonate aqueous solution (50 mL) and extracted with DCM (30 mL × 3). The organic phases were combined and concentrated under vacuum. The residue was purified by SGC (0 to 15% EtOAc in PE) to obtain 3-(8-bromo-3-(2,2,2-trifluoroethyl)indolizin-2-yl)propiolaldehyde **Int-2** (5.70 g, 84.3% yield) as a gray solid. LCMS calculated for C₁₃H₈BrF₃NO [M+H]⁺: m/z = 330.0/332.0; found: 330.2/332.2; ¹H NMR (400 MHz, DMSO-*d*₆) *δ* 9.47 (s, 1H), 8.49 (d, *J* = 7.1 Hz, 1H), 7.28 (d, *J* = 7.1 Hz, 1H), 6.90 (s, 1H), 6.78 (t, *J* = 7.1 Hz, 1H), 4.31 (q, *J* = 10.7 Hz, 2H).

### Cpd-C: 4-((3-(8-(((3S,4R)-3-Fluoro-1-methylpiperidin-4-yl)amino)-3-(2,2,2-trifluoroethyl)indolizin-2-yl)prop-2-yn-1-yl)amino)-3-methoxy-N-methylbenzamide

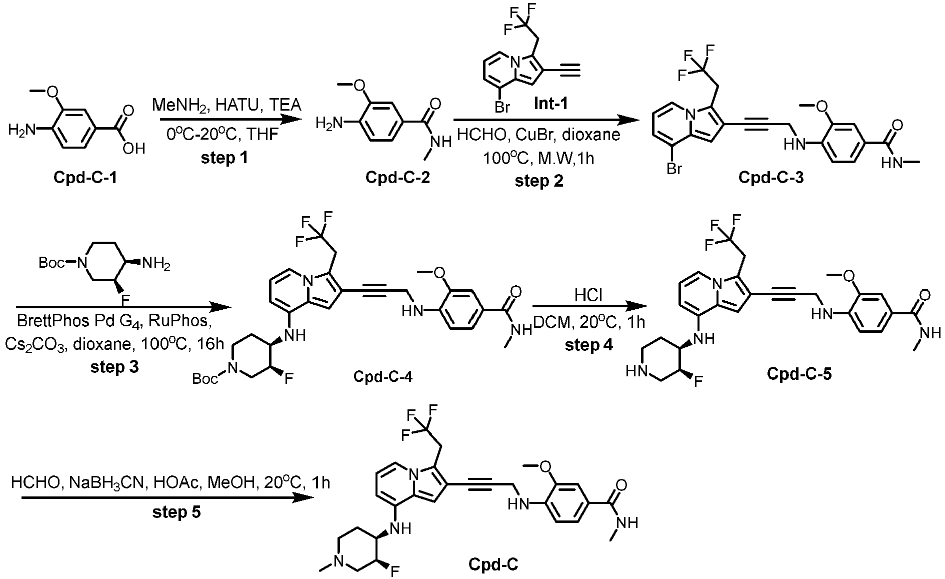

### Step 1: 4-Amino-3-methoxy-N-methylbenzamide

HATU (18.8 g, 49.5 mmol) was added to a solution of 4-amino-3-methoxybenzoic acid **Cpd-C-1** (7.52 g, 45.0 mmol), methylamine solution (6.71 g, 54.0 mmol, 25% content), and TEA (18.8 mL, 135 mmol) in THF (45 mL) at 0°C. The reaction mixture was stirred at 20°C for 2 hours. The resulting reaction mixture was concentrated under reduced pressure. The residue was purified by SGC (0 to 4% MeOH in DCM) to obtain 4-amino-3-methoxy-*N-*methylbenzamide **Cpd-C-2** (6.08 g, 75.0% yield) as a white solid. LCMS calculated for C₉H₁₃N₂O₂ [M+H]⁺: m/z = 181.1; found: 181.2.

### Step 2: 4-((3-(8-Bromo-3-(2,2,2-trifluoroethyl)indolizin-2-yl)prop-2-yn-1-yl)amino)-3-methoxy-N-methylbenzamide

Formaldehyde aqueous solution (497 mg, 5.96 mmol, 36% to 38% content) and cuprous bromide (567 mg, 3.97 mmol) were added to a mixture of 8-bromo-2-ethynyl-3-(2,2,2-trifluoroethyl)indolizine **Int-1** (600 mg, 1.99 mmol) and 4-amino-3-methoxy-*N-*methylbenzamide **Cpd-C-2** (716 mg, 3.97 mmol) in 1,4-dioxane (12 mL). The reaction mixture was stirred under microwave irradiation at 100°C for 1 hour. The residue was purified by SGC (0 to 30% EtOAc in DCM) to obtain 4-((3-(8-bromo-3-(2,2,2-trifluoroethyl)indolizin-2-yl)prop-2-yn-1-yl)amino)-3-methoxy-*N*-methylbenzamide **Cpd-C-3** (480 mg, 48.9% yield) as a yellow solid. LCMS calculated for C₂₂H₂₀BrF₃N₃O₂ [M+H]⁺: m/z = 494.1/496.1; found: 494.2/496.2.

### Step 3: tert-Butyl (3S,4R)-3-fluoro-4-((2-(3-((2-methoxy-4-(methylcarbamoyl)phenyl)amino)prop-1-yn-1-yl)-3-(2,2,2-trifluoroethyl)indolizin-8-yl)amino)piperidine-1-carboxylate

Under an argon atmosphere, a mixture of 4-((3-(8-bromo-3-(2,2,2-trifluoroethyl)indolizin-2-yl)prop-2-yn-1-yl)amino)-3-methoxy-*N*-methylbenzamide **Cpd-C-3** (1.35 g, 2.73 mmol), *tert*-butyl (3*S*,4*R*)-4-amino-3-fluoropiperidine-1-carboxylate (1.19 g, 5.46 mmol), BrettPhos Pd G4 (0.50 g, 0.55 mmol), Ruphos (0.25 g, 0.55 mmol), and cesium carbonate (1.78 g, 5.46 mmol) in 1,4-dioxane (50 mL) was stirred at 100°C for 16 hours. The reaction mixture was diluted with EtOAc (200 mL), washed with water (100 mL) and saturated brine (100 mL), dried over anhydrous Na₂SO₄, filtered, and concentrated under reduced pressure. The residue was purified by SGC (0 to 50% EtOAc in DCM) to obtain *tert*-butyl (3*S*,4*R*)-3-fluoro-4-((2-(3-((2-methoxy-4-(methylcarbamoyl)phenyl)amino)prop-1-yn-1-yl)-3-(2,2,2-trifluoroethyl)indolizin-8-yl)amino)piperidine-1-carboxylate **Cpd-C-4** (1.00 g, 58.0% yield) as a yellow solid. LCMS calculated for C₃₂H₃₈F₄N₅O₄ [M+H]⁺: m/z = 632.3; found: 632.5.

### Step 4: 4-((3-(8-(((3S,4R)-3-Fluoropiperidin-4-yl)amino)-3-(2,2,2-trifluoroethyl)indolizin-2-yl)prop-2-yn-1-yl)amino)-3-methoxy-N-methylbenzamide

HCl (7.92 mL, 31.7 mmol, 4N in 1,4-dioxane) was added to a solution of *tert*-butyl (3*S*,4*R*)-3-fluoro-4-((2-(3-((2-methoxy-4-(methylcarbamoyl)phenyl)amino)prop-1-yn-1-yl)-3-(2,2,2-trifluoroethyl)indolizin-8-yl)amino)piperidine-1-carboxylate **Cpd-C-4** (1.00 g, 1.58 mmol) in DCM (16 mL) at 0°C. The mixture was warmed to room temperature and stirred for 1 hour. The reaction mixture was concentrated to dryness, diluted with water (30 mL), and added with saturated sodium bicarbonate solution to adjust the pH to 7 to 8. The aqueous phase was extracted with DCM (100 mL × 2). The combined organic phases were dried over anhydrous Na₂SO₄, filtered, and concentrated to obtain 4-((3-(8-(((3*S*,4*R*)-3-fluoropiperidin-4-yl)amino)-3-(2,2,2-trifluoroethyl)indolizin-2-yl)prop-2-yn-1-yl)amino)-3-methoxy-*N-*methylbenzamide **Cpd-C-5** (450 mg, crude product) as a yellow solid. The crude product was used directly in the next reaction step. LCMS calculated for C₂₇H₃₀F₄N₅O₂ [M+H]⁺: m/z = 532.2; found: 532.4.

### Step 5: 4-((3-(8-(((3S,4R)-3-Fluoro-1-methylpiperidin-4-yl)amino)-3-(2,2,2-trifluoroethyl)indolizin-2-yl)prop-2-yn-1-yl)amino)-3-methoxy-N-methylbenzamide

Sodium cyanoborohydride (160 mg, 2.54 mmol) was added to a solution of 4-((3-(8-(((3*S*,4*R*)-3-fluoropiperidin-4-yl)amino)-3-(2,2,2-trifluoroethyl)indolizin-2-yl)prop-2-yn-1-yl)amino)-3-methoxy-*N*-methylbenzamide **Cpd-C-5** (450 mg, 0.85 mmol), acetic acid (0.02 mL, 0.42 mmol), and formaldehyde aqueous solution (212 mg, 2.54 mmol, 36% to 38% content) in MeOH (20 mL) at room temperature, and the reaction mixture was stirred at room temperature for 1 hour. The resulting reaction mixture was dropwise added with saturated sodium bicarbonate solution to adjust the pH to 7 to 8. The aqueous phase was extracted with EtOAc (100 mL × 3). The organic phases were combined, washed with saturated brine (50 mL × 3), dried over anhydrous Na₂SO₄, filtered, and concentrated. The residue was purified by preparative chromatography to obtain 4-((3-(8-(((3*S*,4*R*)-3-fluoro-1-methylpiperidin-4-yl)amino)-3-(2,2,2-trifluoroethyl)indolizin-2-yl)prop-2-yn-1-yl)amino)-3-methoxy-*N-*methylbenzamide **Cpd-C** (119 mg, 25.2% yield) as a white solid. LCMS calculated for C₂₈H₃₂F₄N₅O₂ [M+H]⁺: m/z = 546.2; found: 546.2; ¹H NMR (400 MHz, DMSO-*d*₆) *δ* 8.08 (d, *J* = 4.6 Hz, 1H), 7.57 (d, *J* = 6.9 Hz, 1H), 7.41 (dd, *J* = 8.2, 1.7 Hz, 1H), 7.34 (d, *J* = 1.7 Hz, 1H), 6.91 (s, 1H), 6.74 (d, *J* = 8.3 Hz, 1H), 6.51 (t, *J* = 7.2 Hz, 1H), 5.90 (t, *J* = 6.2 Hz, 1H), 5.85 (d, *J* = 7.5 Hz, 1H), 5.54 (d, *J* = 8.4 Hz, 1H), 4.81 (d, *J* = 49.8 Hz, 1H), 4.23 (d, *J* = 6.2 Hz, 2H), 3.92 (q, *J* = 10.7 Hz, 2H), 3.83 (s, 3H), 3.54 (d, *J* = 27.9 Hz, 1H), 3.02 (t, *J* = 10.5 Hz, 1H), 2.80 (d, *J* = 11.3 Hz, 1H), 2.75 (d, *J* = 4.5 Hz, 3H), 2.28-2.14 (m, 4H), 2.07 (t, *J* = 11.2 Hz, 1H), 1.97 (ddd, *J* = 24.0, 12.0, 3.2 Hz, 1H), 1.67 (dd, *J* = 12.7, 2.7 Hz, 1H).

### Example 1: 5-((3-(8-(((3S,4R)-3-Fluoro-1-methylpiperidin-4-yl)amino)-3-(2,2,2-trifluoroethyl)indolizin-2-yl)prop-2-yn-1-yl)amino)-6-(methoxy-d₃)picolinic acid (Cpd-1, i.e., Compound 1)

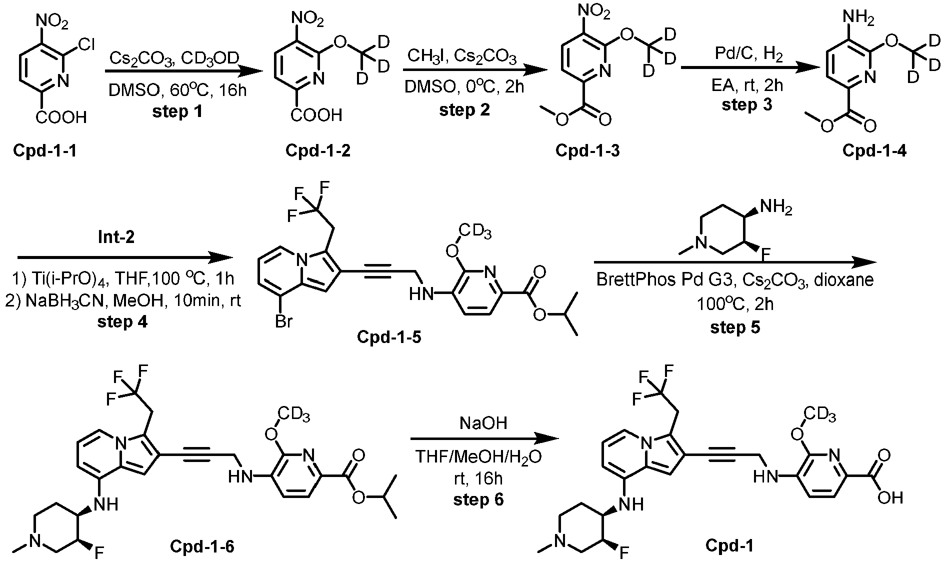

### Step 1: 6-(Methoxy-d₃)-5-nitropicolinic acid

Under a nitrogen atmosphere, a mixture of 6-chloro-5-nitropicolinic acid **Cpd-1-1** (20.0 g, 98.7 mmol), cesium carbonate (96.5 g, 296 mmol), and deuterated methanol (4.27 g, 118 mmol) in DMSO (200 mL) was stirred at 60°C for 16 hours. After the completion of the reaction detected by LCMS, the mixture was diluted with water (100 mL) and added with 1N HCl to adjust the pH to 3. The mixture was filtered to collect the precipitate, and the filter cake was dried under vacuum to obtain 6-(methoxy-*d*₃)-5-nitropicolinic acid **Cpd-1-2** (12.0 g, crude product) as a yellow solid. The crude product was used directly in the next reaction step. LCMS calculated for C₇H₄D₃N₂O₅ [M+H]⁺: m/z = 202.1; found: 201.9.

### Step 2: Methyl 6-(methoxy-d₃)-5-nitropicolinate

Under a nitrogen atmosphere, a mixture of 6-(methoxy-*d*₃)-5-nitropicolinic acid **Cpd-1-2** (12.0 g, 59.7 mmol) and cesium carbonate (58.4 g, 179 mmol) in DMSO (120 mL) was stirred at 0°C for 0.5 hours. Iodomethane (9.32 g, 179 mmol) was then added thereto, and the reaction mixture was stirred at 0°C for 2 hours. After the completion of the reaction detected by LCMS, the reaction mixture was poured into icy ammonium chloride aqueous solution (1000 mL) and extracted with EtOAc (1000 mL × 2). The combined organic layers were dried over anhydrous Na₂SO₄, filtered, and concentrated to obtain a crude product. The residue was purified by SGC (0 to 20% EtOAc in PE) to obtain methyl 6-(methoxy-*d*₃)-5-nitropicolinate **Cpd-1-3**(10.0 g, 70.1% yield) as a yellow solid. LCMS calculated for C₈H₆D₃N₂O₅ [M+H]⁺: m/z = 216.1; found: 215.9.

### Step 3: Methyl 5-amino-6-(methoxy-d₃)picolinate

Methyl 6-(methoxy-*d*₃)-5-nitropicolinate **Cpd-1-3** (10.0 g, 46.5 mmol) was dissolved in EtOAc (100 mL), then Pd/C (9.90 g, 5% content) was added thereto, and the resulting mixture was stirred at room temperature for 2 hours under a hydrogen atmosphere. LCMS detection showed that the starting material was completely consumed and the desired compound was detected. The reaction mixture was filtered and concentrated to obtain methyl 5-amino-6-(methoxy-*d*₃)picolinate **Cpd-1-4** (8.20 g, 90.5% yield) as a yellow solid. LCMS calculated for C₈H₈D₃N₂O₃ [M+H]⁺: m/z = 186.1; found: 186.2; ¹H NMR (400 MHz, DMSO-*d*₆) *δ* 7.53 (d, *J* = 8.0 Hz, 1H), 6.85 (d, *J* = 8.0 Hz, 1H), 5.88 (s, 2H), 3.75 (s, 3H).

### Step 4: Isopropyl 5-((3-(8-bromo-3-(2,2,2-trifluoroethyl)indolizin-2-yl)prop-2-yn-1-yl)amino)-6-(methoxy-d₃)picolinate

Under a nitrogen atmosphere, a solution of 3-(8-bromo-3-(2,2,2-trifluoroethyl)indolizin-2-yl)propiolaldehyde **Int-2** (200 mg, 0.61 mmol), methyl 5-amino-6-(methoxy-*d*₃)picolinate **Cpd-1-4** (135 mg, 0.73 mmol), and tetraisopropyl titanate (517 mg, 1.82 mmol) in THF (2 mL) was stirred at 100°C for 1 hour. The reaction mixture was cooled to 25°C, then added with MeOH (2 mL) and sodium cyanoborohydride (57.1 mg, 0.91 mmol), and stirred for another 10 minutes. LCMS detection showed that the starting material was completely consumed and the desired compound was detected. The reaction mixture was diluted with water (20 mL) and extracted with EtOAc (20 mL × 3). The combined organic layers were washed with saturated brine (50 mL), dried over anhydrous Na₂SO₄, filtered, and concentrated. The residue was purified by SGC (0 to 25% EtOAc in PE) to obtain isopropyl 5-((3-(8-bromo-3-(2,2,2-trifluoroethyl)indolizin-2-yl)prop-2-yn-1-yl)amino)-6-(methoxy-*d*₃)picolinate **Cpd-1-5** (190 mg, 59.4% yield) as a yellow solid. LCMS calculated for C₂₃H₁₉D₃BrF₃N₃O₃ [M+H]⁺: m/z = 527.1; found: 526.9.

### Step 5: Isopropyl 5-((3-(8-(((3S,4R)-3-fluoro-1-methylpiperidin-4-yl)amino)-3-(2,2,2-trifluoroethyl)indolizin-2-yl)prop-2-yn-1-yl)amino)-6-(methoxy-d₃)picolinate

Under a nitrogen atmosphere, a solution of isopropyl 5-((3-(8-bromo-3-(2,2,2-trifluoroethyl)indolizin-2-yl)prop-2-yn-1-yl)amino)-6-(methoxy-*d*₃)picolinate **Cpd-1-5** (170 mg, 0.322mmol), (3*S*,4*R*)-3-fluoro-1-methylpiperidin-4-amine (85.1 mg, 0.64 mmol), Brettphos Pd G3 (58.3 mg, 0.06 mmol), and cesium carbonate (210 mg, 0.65 mmol) in 1,4-dioxane (2 mL) was stirred at 100°C for 2 hours in a sealed tube. LCMS detection showed that the starting material was completely consumed and the desired compound was detected. The reaction mixture was filtered, and the filtrate was concentrated under reduced pressure. The crude product was purified by preparative chromatography to obtain isopropyl 5-((3-(8-(((3*S*,4*R*)-3-fluoro-1-methylpiperidin-4-yl)amino)-3-(2,2,2-trifluoroethyl)indolizin-2-yl)prop-2-yn-1-yl)amino)-6-(methoxy-*d*₃)picolinate **Cpd-1-6** (90.0 mg, 48.1% yield) as a yellow solid. LCMS calculated for C₂₉H₃₁D₃F₄N₅O₃ [M+H]⁺: m/z = 579.3; found: 579.0.

### Step 6: 5-((3-(8-(((3S,4R)-3-Fluoro-1-methylpiperidin-4-yl)amino)-3-(2,2,2-trifluoroethyl)indolizin-2-yl)prop-2-yn-1-yl)amino)-6-(methoxy-d₃)picolinic acid

A mixture of isopropyl 5-((3-(8-(((3*S*,4*R*)-3-fluoro-1-methylpiperidin-4-yl)amino)-3-(2,2,2-trifluoroethyl)indolizin-2-yl)prop-2-yn-1-yl)amino)-6-(methoxy-*d*₃)picolinate **Cpd-1-6** (40.0 mg, 0.07 mmol) and NaOH (8.28 mg, 0.21 mmol) in THF (0.4 mL), MeOH (0.2 mL), and H₂O (0.1 mL) was stirred at room temperature for 16 hours. LCMS detection showed that the starting material was completely consumed and the desired compound was detected. The reaction mixture was concentrated under reduced pressure, diluted with water, then added with diluted HCl to adjust the pH to 5, and filtered. The residue was purified by SGC (0 to 3% MeOH in DCM) to obtain a yellow solid crude product, which was then purified by preparative chromatography to obtain 5-((3-(8-(((3*S*,4*R*)-3-fluoro-1-methylpiperidin-4-yl)amino)-3-(2,2,2-trifluoroethyl)indolizin-2-yl)prop-2-yn-1-yl)amino)-6-(methoxy-*d*₃)picolinic acid **Cpd-1** (5.50 mg, 15.0% yield) as a white solid. LCMS calculated for C₂₆H₂₅D₃F₄N₅O₃ [M+H]⁺: m/z = 537.2; found: 537.0; ¹H NMR (400 MHz, CD₃OD) *δ* 7.64 (d, *J* = 7.3 Hz, 1H), 7.36 (d, *J* = 6.9 Hz, 1H), 6.94 (d, *J* = 7.8 Hz, 1H), 6.56 (s, 1H), 6.42 (t, *J* = 7.2 Hz, 1H), 5.79 (d, *J* = 7.3 Hz, 1H), 4.90 (s, 1H), 4.19 (s, 2H), 3.69 (q, *J* = 10.5 Hz, 2H), 3.62-3.54 (m, 1H), 3.32-3.23 (m, 1H), 3.01 (d, *J* = 12.2 Hz, 1H), 2.62-2.48 (m, 1H), 2.46-2.39 (m, 1H), 2.37 (s, 3H), 2.00-1.87 (m, 2H).

### Example 2: 5-((3-(8-(((3S,4R)-3-Fluoro-1-methylpiperidin-4-yl)amino)-3-(2,2,2-trifluoroethyl)indolizin-2-yl)prop-2-yn-1-yl)amino)-6-methoxypicolinic acid (Cpd-2, i.e., Compound 2)

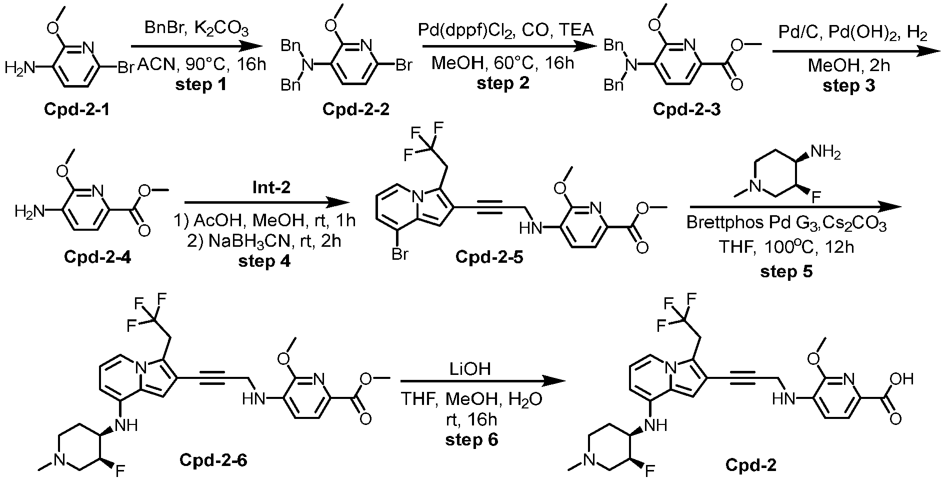

### Step 1: N,N-Dibenzyl-6-bromo-2-methoxypyridin-3-amine

Under a nitrogen atmosphere, a mixture of 6-bromo-2-methoxypyridin-3-amine **Cpd-2-1** (3.00 g, 14.7 mmol), potassium carbonate (8.17 g, 59.1 mmol), and benzyl bromide (7.00 g, 36.9 mmol) in acetonitrile (30 mL) was heated to 90°C and stirred for 16 hours. LCMS detection showed that the starting material was completely consumed and the desired compound was detected. The reaction mixture was filtered and concentrated. The residue was purified by SGC (0 to 10% EtOAc in PE) to obtain *N*,*N*-dibenzyl-6-bromo-2-methoxypyridin-3-amine **Cpd-2-2** (4.00 g, 70.6% yield) as a white solid. LCMS calculated for C₂₀H₂₀BrN₂O [M+H]⁺: m/z = 383.1/385.1; found: 385.3/385.3.

### Step 2: Methyl 5-(dibenzylamino)-6-methoxypicolinate

Under a carbon monoxide atmosphere, a solution of *N*,*N*-dibenzyl-6-bromo-2-methoxypyridin-3-amine **Cpd-2-2** (4.00 g, 10.4 mmol), Pd(dppf)Cl₂ (381 mg, 0.52 mmol), and TEA (2.11 g, 20.8 mmol) in MeOH (100 mL) was heated to 60°C and stirred for 16 hours. LCMS detection showed that the starting material was completely consumed and the desired compound was detected. The reaction mixture was concentrated. The residue was purified by SGC (0 to 10% EtOAc in PE) to obtain methyl 5-(dibenzylamino)-6-methoxypicolinate **Cpd-2-3** (3.00 g, 71.4% yield) as a white solid. LCMS calculated for C₂₂H₂₃N₂O₃ [M+H]⁺: m/z = 363.2; found: 363.4.

### Step 3: Methyl 5-amino-6-methoxypicolinate

Methyl 5-(dibenzylamino)-6-methoxypicolinate **Cpd-2-3** (3.00 g, 8.28 mmol) was dissolved in MeOH (50 mL), then Pd/C (100 mg, 0.83 mmol, 10% content) and Pd(OH)₂ (100 mg, 0.83 mmol, 10% content) were added thereto, and the system was stirred at room temperature for 2 hours under a hydrogen atmosphere. LCMS detection showed that the starting material was completely consumed and the desired compound was detected. The reaction mixture was filtered and concentrated. The residue was purified by SGC (0 to 50% EtOAc in PE) to obtain methyl 5-amino-6-methoxypicolinate **Cpd-2-4** (1.00 g, 66.3% yield) as a white solid. LCMS calculated for C₈H₁₁N₂O₃ [M+H]⁺: m/z = 183.2; found: 183.4.

### Step 4: Methyl 5-((3-(8-bromo-3-(2,2,2-trifluoroethyl)indolizin-2-yl)prop-2-yn-1-yl)amino)-6-methoxypicolinate

A solution of 3-(8-bromo-3-(2,2,2-trifluoroethyl)indolizin-2-yl)propiolaldehyde **Int-2** (200 mg, 0.61 mmol), methyl 5-amino-6-methoxypicolinate **Cpd-2-4** (110 mg, 0.61 mol), and acetic acid (72.7 mg, 1.21 mmol) in MeOH (20 mL) was stirred at room temperature for 2 hours. Sodium cyanoborohydride (76.1 mg, 1.21 mmol) was added portionwise thereto, and the resulting mixture was stirred for another 2 hours. LCMS detection showed that the starting material was completely consumed and the desired compound was detected. The reaction mixture was quenched with saturated sodium bicarbonate solution (10 mL) and extracted with DCM (50 mL × 2). The organic phases were combined, dried over anhydrous Na₂SO₄, filtered, and concentrated. The residue was purified by SGC (0 to 100% DCM in PE) to obtain methyl 5-((3-(8-bromo-3-(2,2,2-trifluoroethyl)indolizin-2-yl)prop-2-yn-1-yl)amino)-6-methoxypicolinate **Cpd-2-5** (200 mg, 66.5% yield) as a yellow solid. LCMS calculated for C₂₁H₁₈BrF₃N₃O₃ [M+H]⁺: m/z = 496.0/498.0; found: 496.3/498.3.

### Step 5: Methyl 5-((3-(8-(((3S,4R)-3-fluoro-1-methylpiperidin-4-yl)amino)-3-(2,2,2-trifluoroethyl)indolizin-2-yl)prop-2-yn-1-yl)amino)-6-methoxypicolinate

Under an argon atmosphere, a mixture of methyl 5-((3-(8-bromo-3-(2,2,2-trifluoroethyl)indolizin-2-yl)prop-2-yn-1-yl)amino)-6-methoxypicolinate **Cpd-2-5** (200 mg, 0.40 mol), (3*S*,4*R*)-3-fluoro-1-methylpiperidin-4-amine (79.9 mg, 0.60 mol), Brettphos Pd G3 (38.7 mg, 0.04 mmol), cesium carbonate (263 mg, 0.80 mol), and Ruphos (37.6 mg, 0.08 mmol) in THF (5 mL) was heated to 100°C and stirred for 12 hours. LCMS detection showed that the starting material was completely consumed and the desired compound was detected. The reaction mixture was filtered and concentrated. The residue was purified by SGC (0 to 10% MeOH in DCM) to obtain methyl 5-((3-(8-(((3*S*,4*R*)-3-fluoro-1-methylpiperidin-4-yl)amino)-3-(2,2,2-trifluoroethyl)indolizin-2-yl)prop-2-yn-1-yl)amino)-6-methoxypicolinate **Cpd-2-6** (100 mg, 40.7% yield) as a yellow oil. LCMS calculated for C₂₇H₃₀F₄N₅O₃ [M+H]⁺: m/z = 548.2; found: 548.5.

### Step 6: 5-((3-(8-(((3S,4R)-3-Fluoro-1-methylpiperidin-4-yl)amino)-3-(2,2,2-trifluoroethyl)indolizin-2-yl)prop-2-yn-1-yl)amino)-6-methoxypicolinic acid

Methyl 5-((3-(8-(((3*S*,4*R*)-3-fluoro-1-methylpiperidin-4-yl)amino)-3-(2,2,2-trifluoroethyl)indolizin-2-yl)prop-2-yn-1-yl)amino)-6-methoxypicolinate **Cpd-2-6** (320 mg, 0.854 mmol) was dissolved in MeOH (4 mL) and THF (4 mL), then lithium hydroxide aqueous solution (1 mL, 5.84 mmol) was added thereto, and the resulting mixture was stirred at room temperature for 16 hours. LCMS detection showed that the starting material was completely consumed and the desired compound was detected. The reaction mixture was concentrated to obtain 5-((3-(8-(((3*S*,4*R*)-3-fluoro-1-methylpiperidin-4-yl)amino)-3-(2,2,2-trifluoroethyl)indolizin-2-yl)prop-2-yn-1-yl)amino)-6-methoxypicolinic acid (320 mg, crude product) as a yellow solid. 100 mg of the crude product was purified by preparative chromatography to obtain 5-((3-(8-(((3*S*,4*R*)-3-fluoro-1-methylpiperidin-4-yl)amino)-3-(2,2,2-trifluoroethyl)indolizin-2-yl)prop-2-yn-1-yl)amino)-6-methoxypicolinic acid **Cpd-2** (32.5 mg, 33.4% yield) as a white solid. LCMS calculated for C₂₆H₂₈F₄N₅O₃ [M+H]⁺: m/z = 534.2; found: 534.5; ¹H NMR (400 MHz, DMSO-*d*₆) δ 12.09 (br, 1H), 7.62 (d, *J* = 8.0 Hz, 1H), 7.55 (d, *J* = 6.8 Hz, 1H), 6.98 (d, *J* = 8.1 Hz, 1H), 6.89 (s, 1H), 6.56 (t, *J* = 6.1 Hz, 1H), 6.49 (t, *J* = 7.2 Hz, 1H), 5.83 (d, *J* = 7.5 Hz, 1H), 5.53 (d, *J* = 8.4 Hz, 1H), 4.80 (d, *J* = 49.6 Hz, 1H), 4.23 (d, *J* = 6.1 Hz, 2H), 3.98-3.85 (m, 5H), 3.62-3.44 (m, 1H), 3.01 (t, *J* = 10.6 Hz, 1H), 2.79 (d, 1H), 2.33-2.22 (m, 1H), 2.17 (s, 3H), 2.07 (t, 1H), 2.03-1.89 (m, 1H), 1.72-1.61 (m, 1H).

### Biological Evaluation

### I. Biochemical assay: Test of compounds promoting the binding of p53 Y220C mutant with DNA

Homogeneous time-resolved fluorescence (HTRF) assay was used to measure the reactivation effect of compounds on the p53 Y220C mutant. Recombinant His-tagged p53 Y220C (94-312) used for HTRF assays was expressed in *E. coli* and purified to 90% purity using a Ni-NTA column. The biotin-labeled DNA used for HTRF assays was synthesized by Pharmaron Inc, with the specific sequence of 5'-ATTAGGCATGTCTAGGCATGTCTAGG-3'.

The binding of recombinant His-tagged p53 Y220C protein with biotin-labeled DNA was measured using fluorescence resonance energy transfer (FRET). For FRET assays, the binding between the p53 mutant and the DNA sequences was measured by detecting the fluorescence of the interaction between Eu-conjugated anti-His antibody (Cisbio, 61HI2KLA) and d2-conjugated streptavidin (Cisbio, 610SADLF) bound to a biotin-labeled DNA molecule. Compounds were prepared as 2 mM stock solutions and serially diluted 1:3 in DMSO to obtain 10 concentrations. Each compound at 200× working concentration was sequentially diluted to 4× working concentration. 4 µL of compound solution was transferred row-by-row to a 384-well assay plate using Echo, with 2 replicates per column. Subsequently, 4 µL of p53 solution was added to the assay plate, followed by 4 µL of biotinylated DNA solution, and then 4 µL of assay solution (His-Eu antibody and streptavidin-d2) was added to each well. The assay plate was incubated overnight in the dark. Fluorescence was read on a BMG microplate reader (BMG LABTECH).

The ratio for each well was calculated (ratio_665 nm/615 nm - ratio_background). The activity (%) was calculated as follows:
$C = \left(Ave_Ac-Ave_Ba\right) / \left(Ave_Dc-Ave_Bd\right)$
$R data = \left(A-Ave_Ba-C\times D\right) / \left(D-Ave_Bd\right) \times \left(Ave_Dc-Ave_Bd\right)$
$Activity \left(%\right) = R data / Ave _ VC \times 100 .$
A: fluorescence intensity of the sample at 665 nm;
D: fluorescence intensity of the sample at 615 nm;
Ba: fluorescence intensity of the plate background at 665 nm;
Bd: fluorescence intensity of the plate background at 615 nm;
Dc: fluorescence intensity of HIS-Eu background at 615 nm;
Ac: fluorescence intensity of streptavidin-d2 background at 665 nm;
VC: DMSO treatment group.

SC₁₅₀ was calculated using GraphPad 8.0 to fit the logarithm of activity (%) versus compound concentration to a nonlinear regression (dose response-variable slope).

**Table 1: Activity of compounds promoting the binding of p53 Y220C mutant with DNA**

| Compound No. | SC₁₅₀ (nM) |
|---|---|
| Cpd-A | 10 |
| Cpd-B | 15 |
| Cpd-C | 4.6 |
| Cpd-1 | 0.4 |
| Cpd-2 | 0.5 |

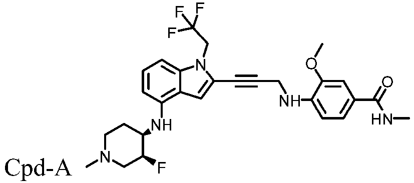

is selected from WO2021061643A1. 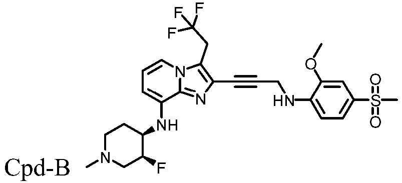 is selected from WO2022213975A1.

The above data show that the representative compounds of the present disclosure have good activity for promoting the binding of p53 Y220C mutants with DNA, and are obviously superior to the reference compounds.

### II. Cellular assay: Test of anti-proliferation effect on NUGC-3 cells

The anti-proliferation effect was assessed against the human gastric adenocarcinoma cell line based on the activity of dehydrogenase in cells, which showed good correlation with the number of living cells. Cell line NUGC-3 (JCRB, JCRB0822) was cultured in RPMI 1640 medium (Invitrogen, 11875-085) supplemented with 10% (v/v) fetal bovine serum (BI, 04-002-1A), according to the standard instructions of the American Type Culture Collection. The cell line was authenticated by short tandem repeat profiling.

In the proliferation assay, 1000 NUGC-3 cells were seeded in a 96-well flat clear bottom, TC-treated plate (Corning, 3903) in 200 µL of medium per well, and allowed to recover overnight in culture medium. Compounds were added with increasing concentration, with DMSO treatment serving as a positive control. After 5 days of treatment, the plate was equilibrated to room temperature for 30 minutes, and the number of living cells was measured by the addition of the Cell Counting Kit-8 reagent (MCE, HY-K0301). Absorbance was measured on an EnSpire microplate reader (PerkinElmer). The relative viability of each group was presented as the percentage change relative to the positive control group and then fit to a four-parameter logit non-linear curve using the program XLFit (IDBS).

**Table 2: Anti-proliferation activity of compounds on NUGC-3 cells**

| Compound No. | NUGC-3 IC₅₀ (µM) |
|---|---|
| Cpd-A | 0.78 |
| Cpd-B | 0.36 |
| Cpd-C | 0.23 |
| Cpd-1 | 0.10 |
| Cpd-2 | 0.12 |

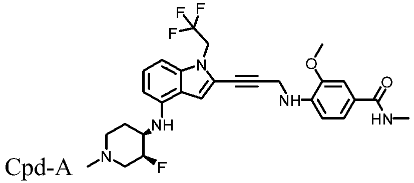

is selected from WO2021061643A1. 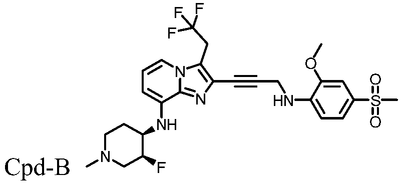 is selected from WO2022213975A1.

The above data show that the representative compounds of the present disclosure have good inhibitory activity on the proliferation of NUGC-3 cells, and are obviously superior to the reference compounds.

### III. Evaluation of drug-drug interactions related to metabolic enzymes

### (i) Test of the inhibitory activity on CYP isoenzymes at a single concentration

1. Experimental purpose: To test the inhibitory effect of compounds on CYP isoenzymes at a single concentration.
2. Experimental instrument and reagents:

### Liver microsome:

| Species | Gender | Supplier | Cat. No. | Batch | Storage |
|---|---|---|---|---|---|
| Human | Mix | Corning | 452117 | 38298 | -80°C |

### Reagents:

| Compound | Supplier | Cat. No. | Storage |
|---|---|---|---|
| Terfenadine | Sigma-Aldrich | T9652 | 4°C |
| Tolbutamide | Sigma-Aldrich | T0891 | 4°C |
| Potassium hydrogen phosphate | Sinopharm | 20032117 | Room temperature |
| Reduced nicotinamide adenine dinucleotide phosphate | Shanghai ABCONE Biotechnology Co., Ltd. | N99640-100MG | -20°C |
| Phenacetin | Sigma-Aldrich | 101690303 | Room temperature |
| Diclofenac | Sigma-Aldrich | D6899-10G | Room temperature |
| Mephenytoin | GlpBio | GC14486 | -20°C |
| Dextromethorphan | Sigma-Aldrich | D9684-5G | Room temperature |
| Midazolam | National Institute for Food and Drug Control | PVJT-0H9Z | Room temperature |
| Testosterone | Adamas Reagent | 171265 | Room temperature |
| Bupropion | Cayman Chemical Company | 10488 | -20°C |
| Amodiaquine | AbMole | M5412 | -20°C |
| β-Naphthoflavone | Sigma-Aldrich | N5757-1G | 4°C |
| Sulfaphenazole | Sigma-Aldrich | S0758-1G | 4°C |
| Benzylnirvanol | Shanghai Yuanye Bio-Technology Co., Ltd. | Y43177-5 mg | Room temperature |
| Quinidine | Internation-Laboratory-USA | 1311468-5G | Room temperature |
| Ketoconazole | Sigma-Aldrich | K1003-100MG | 4°C |

### 3. Experimental methods:

8.71 g of K₂HPO₄ was dissolved in 950 mL of water. The mixture was added with HCl to adjust the pH to 7.4 and brought to a final volume of 1000 mL with water. The buffer was filtered through a 0.45 µm filter and stored in a refrigerator at 4°C for future use. Terfenadine/tolbutamide (1 mg/mL each) stock solutions were prepared with DMSO. The above stock solution was diluted with acetonitrile to prepare a quenching solution containing 5/10 ng/mL (terfenadine/tolbutamide).

### Positive control working solutions:

| CYP isoenzyme | Inhibitor | Stock solution conc. (mM) | Working solution conc. (mM) | Incubation conc. (µM) | Stock solution volume (µL) | Total volume (µL) | Format |
|---|---|---|---|---|---|---|---|
| 1A2 | β-Naphthoflavone | 10 | 2 | 10 | 10 | 50 | 5 in 1 |
| 2C9 | Sulfaphenazole | 10 | 2 | 10 | 10 | | |
| 2C19 | Benzylnirvanol | 5 | 1 | 5 | 10 | | |
| 2D6 | Quinidine | 10 | 2 | 10 | 10 | | |
| 3A4/5 | Ketoconazole | 10 | 2 | 10 | 10 | | |
| | Ketoconazole | 2 | 2 | 10 | 50 | 50 | Single |

### Substrate working solutions:

| CYP isoenzyme | Substrate | Stock solution conc. (mM) | Working solution conc. (mM) | Incubation conc. (µM) | Stock solution volume (µL) | Total volume (µL) | Format |
|---|---|---|---|---|---|---|---|
| 1A2 | Phenacetin | 250 | 50 | 50 | 2 | 10 | 5 in 1 |
| 2C9 | Diclofenac | 50 | 10 | 10 | 2 | | |
| 2C19 | Mephenytoin | 225 | 45 | 45 | 2 | | |
| 2D6 | Dextromethorphan | 25 | 5 | 5 | 2 | | |
| 3A4/5 | Midazolam | 12.5 | 2.5 | 2.5 | 2 | | |
| | Testosterone | 50 | 50 | 50 | 5 | 5 | Single |

Liver microsomes were thawed in a water bath at 37°C prior to use. Liver microsome working solution (final concentration of 0.5 mg/mL) was prepared. A 5 mM NADPH working solution was prepared in phosphate-buffered saline. The above stock solution was prepared into a 2 mM working solution with DMSO.

238.5 µL of liver microsome working solution was added to a 1.1 mL tube, followed by 1.5 µL of test compound/control working solution/DMSO, and mixed well by pipetting several times. The mixture was pre-incubated in a water bath with shaking at 37°C for 5 minutes. After pre-incubation, 60 µL of NADPH working solution was added and mixed well by pipetting several times. The mixture was incubated in a water bath with shaking at 37°C for 10 minutes. Immediately after incubation, 500 µL of quenching solution was added and vortexed for 1 minute. All samples were centrifuged at 4,000 rpm for 15 minutes at 4°C. 300 µL of the supernatant was aliquoted for further LC-MS/MS analysis.

### 4. Data processing and results:

Inhibition rate (% inhibition) was calculated using metabolite formation of the test compound or the control compound compared to the matrix control.

**Table 3: Inhibitory activity of compounds on CYP isoenzymes***

| Compound No. | Inhibition rate (%)* | | | | | |
|---|---|---|---|---|---|---|
| | CYP1A2 | CYP2C9 | CYP2C19 | CYP2D6 | CYP3A4/5 (Midazolam) | CYP3A4/5 (Testosterone) |
| Cpd-A | 12 | 38 | 42 | 5.5 | 83 | ND |
| Cpd-B | 0.56 | 21 | 31 | 3.6 | 61 | 75 |
| Cpd-C | 18 | 43 | 67 | 9.5 | 79 | ND |
| Cpd-1 | 6.3 | NI | 5.5 | 2.6 | 3.4 | NI |
| Cpd-2 | 1.2 | NI | 1.9 | 0.73 | NI | 1.7 |

| | | | | | | |
|---|---|---|---|---|---|---|
| *Final compound concentration: 10 µM; ND: not detected; NI: no inhibition. | | | | | | |

The above data show that the representative compounds of the present disclosure have relatively weak inhibitory activity on CYP isoenzymes, and the risk of drug-drug interaction is significantly lower than that of the reference compounds.

### (ii) Determination of the multi-concentration inhibitory activity curve of CYP3A4/5 enzymes

1. Experimental purpose: To determine the concentration inhibitory activity curve of compounds on CYP3A4/5 enzymes.
2. Experimental instrument and materials:

### Liver microsome:

| Species | Gender | Supplier | Cat. No. | Batch | Storage |
|---|---|---|---|---|---|
| Human | Mix | Corning | 452117 | 38298 | -80°C |

### Instrument and equipment:

| Equipment | Model | Supplier |
|---|---|---|
| Mass spectrometer | -- | AB SCIEX |
| Centrifuge | X3R | Thermo Scientific |
| Analytical balance | TB-25 | Denver Instrument |
| Shaker | TS-2 orbital shaker | Kylin-Bell Lab Instruments |
| Water bath | THZ-82 | Changzhou Jintan Honghua Instrument Factory |

### Reagents:

| Compound | Supplier | Cat. No. | Storage |
|---|---|---|---|
| Terfenadine | Sigma-Aldrich | T9652 | 4°C |
| Tolbutamide | Sigma-Aldrich | T0891 | 4°C |
| Potassium hydrogen phosphate | Sinopharm | 20032117 | Room temperature |
| Reduced nicotinamide adenine dinucleotide phosphate | Shanghai ABCONE Biotechnology Co., Ltd. | N99640-100MG | 20°C |
| Midazolam | National Institute for Food and Drug Control | PVJT-0H9Z | Room temperature |
| Testosterone | Adamas Reagent | 171265 | Room temperature |
| Ketoconazole | Sigma-Aldrich | K1003-100MG | 4°C |

### 3. Experimental methods:

8.71 g of K₂HPO₄ was dissolved in 950 mL of water. The mixture was added with HCl to adjust the pH to 7.4 and brought to a final volume of 1000 mL with water. The buffer was filtered through a 0.45 µm filter and stored in a refrigerator at 4°C for future use. Terfenadine/tolbutamide (1 mg/mL each) stock solutions were prepared with DMSO. The above stock solution was diluted with acetonitrile to prepare a quenching solution containing 5/10 ng/mL (terfenadine/tolbutamide).

### Positive control working solutions:

| CYP isoenzyme | Inhibitor | Stock solution conc. (mM) | Working solution conc. (mM) | Incubation conc. (µM) | Stock solution volume (µL) | Total volume (µL) |
|---|---|---|---|---|---|---|
| 3A4/5 | Ketoconazole^{a} | 10 | 2 | 10 | 10 | 50 |
| | Ketoconazole^{b} | 2 | 2 | 10 | 50 | 50 |

| | | | | | | |
|---|---|---|---|---|---|---|
| a: midazolam as substrate; b: testosterone as substrate. | | | | | | |

### Substrate working solutions:

| CYP isoenzyme | Substrate | Stock solution conc. (mM) | Working solution conc. (mM) | Incubation conc. (µM) | Stock solution volume (µL) | Total volume (µL) |
|---|---|---|---|---|---|---|
| 3A4/5 | Ketoconazole | Midazolam | 12.5 | 2.5 | 2.5 | 2 |
| | Ketoconazole | Testosterone | 50 | 50 | 50 | 5 |

Liver microsomes were thawed in a water bath at 37°C prior to use. Liver microsome working solution (final concentration of 0.5 mg/mL) was prepared. A 5 mM NADPH working solution was prepared in phosphate-buffered saline. The above stock solution was prepared into a 2 mM working solution with DMSO. The above stock solution was diluted with acetonitrile (4-fold dilution, 6 non-zero concentrations).

238.5 µL of liver microsome working solution was added to a 1.1 mL tube, followed by 1.5 µL of test compound/control working solution/DMSO, and mixed well by pipetting several times. The mixture was pre-incubated in a water bath with shaking at 37°C for 5 minutes. After pre-incubation, 60 µL of NADPH working solution was added and mixed well by pipetting several times. The mixture was incubated in a water bath with shaking at 37°C for 10 minutes. Immediately after incubation, 500 µL of quenching solution was added and vortexed for 1 minute. All samples were centrifuged at 4,000 rpm for 15 minutes at 4°C. 300 µL of the supernatant was aliquoted for further LC-MS/MS analysis.

### 4. Data processing and results:

Inhibition rate (% inhibition) was calculated using metabolite formation of the test compound or the control compound compared to the matrix control. The half-maximal inhibitory concentration (IC₅₀) was calculated using Graph Pad Prism.

**Table 3: Inhibitory activity of compounds on CYP3A4/5 enzymes**

| Compound No. | CYP3A4/5 IC₅₀ (µM)^{a} | CYP3A4/5 IC₅₀ (µM)^{b} |
|---|---|---|
| Cpd-A | 2.3 | 0.89 |
| Cpd-B | ND | ND |
| Cpd-C | 3.8 | ND |
| Cpd-1 | >50 | >50 |
| Cpd-2 | >50 | >50 |

| | | |
|---|---|---|
| a: midazolam as substrate; b: testosterone as substrate; ND: not detected. | | |

The above experiments indicate that the representative compounds of the present disclosure have relatively weak inhibitory activity on CYP3A4/5 enzymes, and are obviously superior to the reference compounds.

### IV. Cardiac safety evaluation: Test of the inhibitory activity on hERG potassium channel

### 1. Experimental purpose:

The manual patch-clamp technique was used to evaluate whether test compounds have potential inhibitory effects on the voltage-gated hERG potassium channel. This experiment was conducted to evaluate the effect of compounds in five concentrations on hERG channel current, obtaining a dose-effect curve and calculating the IC₅₀.

### 2. Experimental instrument and reagents:

| Experimental instrument or materials | Supplier (Cat. No.) |
|---|---|
| 6 cm cell culture dish | Shanghai Yes Service Biotech, Inc. (150462) |
| 3.5 cm cell culture dish | Shanghai Yes Service Biotech, Inc. (150460) |
| Dialyzed fetal bovine serum | Shanghai Bohan Biotechnology Co., Ltd. (BS-0005-500) |
| DMSO | Beijing Solarbio Science & Technology Co., Ltd. (D8371) |
| DMEM medium | Thermo Fisher Scientific (China) Co., Ltd. (10569044) |
| HEPES | Thermo Fisher Scientific (China) Co., Ltd. (15630080) |
| Trypsin | Thermo Fisher Scientific (China) Co., Ltd. (12604) |
| Phosphate-buffered saline (without calcium and magnesium ions) | Beijing Solarbio Science & Technology Co., Ltd. (D1040) |
| Penicillin-streptomycin solution | Thermo Fisher Scientific (China) Co., Ltd. (15140122) |
| MEM non-essential amino acid solution | Thermo Fisher Scientific (China) Co., Ltd. (11140050) |
| Geneticin (G418) | Thermo Fisher Scientific (China) Co., Ltd. (11811031) |
| Blasticidin | Thermo Fisher Scientific (China) Co., Ltd. (R21001) |
| Polylysine | Thermo Fisher Scientific (China) Co., Ltd. (P4832) |
| Dofetilide | Beijing Express Technology Co., Ltd. (D525700) |
| Doxycycline | Sigma-Aldrich (Shanghai) Trading Co., Ltd. (D9891) |
| Balance | Mettler-Toledo International Inc. (ME104E) |
| pH meter | Mettler-Toledo International Inc. (S220-USP) |
| Carbon dioxide incubator | Thermo Fisher Scientific (China) Co., Ltd. (Thermo371) |
| Glass microelectrode puller | NARISHIGE, Japan (PC-10 Puller) |
| Micromanipulator | Qingdao Sources Optics Co., Ltd. (MC1000e) |
| Amplifier | HEKA, Germany (EPC10) |
| Microscope | Olympus (China) Co., Ltd. (IX-71/73) |
| Perfusion system | ALA Scientific, USA (VM8 gravity-flow delivery system) |
| Vacuum pump | Chemvak, Germany (V300) |
| Osmometer | YASN Lab Technologies (Osmo310) |

### 3. Experimental methods:

Cell line and cell culture: HEK293 cell line stably expressing the hERG ion channel (Cat. No.: K1236) was purchased from Invitrogen. The cell line was cultured in medium containing 85% DMEM, 10% dialyzed fetal bovine serum, 0.1 mM non-essential amino acid solution, 100 U/mL penicillin-streptomycin solution, 25 mM HEPES, 5 µg/mL blasticidin, and 400 µg/mL geneticin. When the cell density grew to 40% to 80% of the bottom area of the culture dish, the cells were digested with trypsin and passaged three times per week. Prior to the experiment, the cells were cultured at a density of 5 × 10⁵ cells per 3.5 cm culture dish, induced with 1 µg/mL doxycycline for 48 hours, followed by digestion, and seeded onto glass slides for subsequent manual patch-clamp experiments.

Solution preparation: Extracellular fluid (in mM): 132 sodium chloride, 4 potassium chloride, 3 calcium chloride, 0.5 magnesium chloride, 11.1 glucose, and 10 HEPES (pH adjusted to 7.35 with sodium hydroxide). Intracellular fluid (in mM): 10 EGTA, 10 HEPES, 10 potassium chloride, 10 sodium chloride, and 110 potassium fluoride (pH adjusted to 7.2 with potassium hydroxide). Osmolality of the solutions was controlled between 280 and 300 mOsmol/kg. The solutions were filtered and stored at 4°C prior to use.

Preparation of test compound solutions: Following the standard operating procedures, test compounds were dissolved in DMSO and prepared into a stock solution with a final concentration of 10 mM. The stock solution was serially diluted 1:3 in DMSO as the solvent to obtain three intermediate solutions with concentrations of 3.33, 1.11, and 0.37 mM. Prior to the experiment, the test compound intermediate solutions were further diluted 1:1000 with extracellular fluid to prepare a series of working solutions. The 30 µM working solution was prepared by diluting the 10 mM stock solution 1:333.33, resulting in final concentrations of 30, 10, 3.33, 1.11, and 0.37 µM. The content of DMSO in the working solution was 0.1 to 0.3% (v/v). Five working solutions with concentrations of 30, 10, 3.33, 1.11, and 0.37 µM were used to determine the potential inhibitory effect of compounds on hERG channels and to fit the dose-effect curve and calculate the IC₅₀.

Experimental procedure: A coverslip loaded with HEK293 cells in a culture dish was placed in the perfusion chamber of the microscope stage. Suitable cells were positioned at the center of the field of view under an Olympus IX71 or IX73 inverted microscope. Using a 10× objective lens, the tip of the glass electrode was located and positioned at the center of the field of view. The electrode was then lowered using a micromanipulator, while the coarse focus screw was adjusted to slowly bring the electrode closer to the cells. On the point of approaching the cells, the objective lens was switched to a ×40 objective lens for observation, and the electrode was gradually brought closer to the cell surface using a micromanipulator for fine adjustment. Negative pressure was applied to form a seal between the electrode tip and the cell membrane with a resistance greater than 1 GΩ. In voltage-clamp mode, the transient capacitive current (Cfast) was compensated. Brief negative pressure was then applied repeatedly to rupture the membrane, eventually forming a whole-cell recording mode. Under the condition that the membrane potential was clamped at -60 mV, the slow capacitive current (Cslow), the cell membrane capacitance (Cm), and the access resistance (Ra) were compensated. After the cells were stable, the clamping voltage was changed to -90 mV, the sampling frequency was set at 20 kHz, and the filtering frequency was 10 kHz. The condition for detecting the leakage current was that the clamping voltage changed to -80 mV and the time duration was 500 ms. The hERG current was measured by applying a 4.8-second depolarizing command voltage to bring the membrane potential from -80 mV to +30 mV, followed by a 5.2-second repolarizing voltage to lower the membrane potential to -50 mV to remove channel inactivation, thereby allowing the hERG tail current to be observed. The peak value of the tail current was the magnitude of the hERG current. The hERG current for the test compound was continuously recorded for 120 seconds before administration to evaluate the stability of the hERG current generated by the test cells. Only stable cells within the acceptable range of the evaluation criteria were allowed to proceed to the subsequent compound testing. Test of the inhibitory effect of the test compound on hERG current: First, the hERG current measured in extracellular fluid containing 0.1% DMSO was used as the measurement baseline. After the hERG current remained stable for at least 5 minutes, the solution containing the test compound was sequentially perfused around the cells from a low concentration to a high concentration. After each perfusion was completed, approximately 5 minutes were allowed for the compound to fully act on the cells, and hERG currents were synchronously recorded. Once the recorded current stabilized, the last five hERG current values were recorded and averaged as the final current value at a specific concentration. After compound testing, 450 nM dofetilide was applied to the same cell to completely inhibit the current, serving as a positive control for the cell. Simultaneously, the positive compound dofetilide was tested using the same patch-clamp system before and after the test compound experiment to ensure the reliability and sensitivity of the entire testing system. The above test steps were repeated on two separate test cells (n = 2).

### 4. Data processing and results:

Data quality control criteria: The initial seal resistance was greater than 1 GΩ; The series resistance was less than 15 MΩ with a voltage error below 5 mV; The leakage current at the test voltage was less than 50% of the current value under the same condition; The tail current was greater than the pre-pulse plateau current, with an initial tail current value exceeding 250 pA; The access resistance (Ra) was less than 15 MΩ; The decay rate of the tail current per minute was less than 2.5%.

Data analysis: Only data meeting the above criteria were analyzed according to the following steps.

Note: Data were exported from PatchMaster software.

After perfusion with blank solvent or compound solutions, the average of five consecutive stable current values was calculated as "tail current_{blank}" and "tail current_{compound}", respectively.

The percentage of current inhibition was calculated using the following formula. $tail current inhibition rate = \left(1-\frac{tail {current}_{compound} - tail {current}_{positive ctrl}}{tail {current}_{blank} - tail {current}_{positive ctrl}}\right) \times 100$

Dose-effect curves were fitted using GraphPad Prism 8.0 software and IC₅₀ values were calculated.

**Table 4: Inhibitory activity of compounds on hERG potassium channel**

| Compound No. | hERG IC₅₀ (µM)^{a} |
|---|---|
| Cpd-A | 19 |
| Cpd-B | 5.5 |
| Cpd-C | 1.4 |
| Cpd-1 | >30 |
| Cpd-2 | >30 |

| | |
|---|---|
| a: ND indicates not detected. | |

The above data show that the representative compounds of the present disclosure have a lower risk of potential cardiotoxicity caused by hERG inhibition, and their safety is obviously superior to that of the reference compounds.

## Claims

1. A compound of formula (I), an isotope-labeled compound thereof, a solvate thereof, a pharmaceutically acceptable salt thereof, a solvate of the pharmaceutically acceptable salt thereof, or a prodrug thereof; 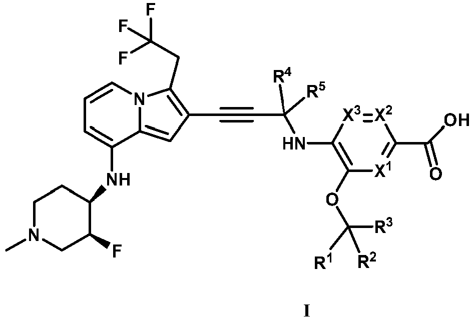 wherein
X¹, X², and X³ are each independently selected from CH and N;
R¹, R², R³, R⁴, and R⁵ are each independently selected from hydrogen and deuterium.

2. The compound of formula (I), the isotope-labeled compound thereof, the solvate thereof, the pharmaceutically acceptable salt thereof, the solvate of the pharmaceutically acceptable salt thereof, or the prodrug thereof according to claim 1, wherein
X¹ is N; X² is CH or N; X³ is CH.

3. The compound of formula (I), the isotope-labeled compound thereof, the solvate thereof, the pharmaceutically acceptable salt thereof, the solvate of the pharmaceutically acceptable salt thereof, or the prodrug thereof according to claim 1, wherein
X¹ is N; X² and X³ are CH.

4. The compound of formula (I), the isotope-labeled compound thereof, the solvate thereof, the pharmaceutically acceptable salt thereof, the solvate of the pharmaceutically acceptable salt thereof, or the prodrug thereof according to any one of claims 1 to 3, wherein
R¹, R², R³, R⁴, and R⁵ are each independently selected from hydrogen and deuterium, and at least one of them is deuterium.

5. The compound of formula (I), the isotope-labeled compound thereof, the solvate thereof, the pharmaceutically acceptable salt thereof, the solvate of the pharmaceutically acceptable salt thereof, or the prodrug thereof according to any one of claims 1 to 3, wherein
R¹, R², and R³ are deuterium;
R⁴ and R⁵ are hydrogen.

6. The compound of formula (I), the isotope-labeled compound thereof, the solvate thereof, the pharmaceutically acceptable salt thereof, the solvate of the pharmaceutically acceptable salt thereof, or the prodrug thereof according to any one of claims 1 to 3, wherein
R¹, R², and R³ are hydrogen;
R⁴ and R⁵ are deuterium.

7. The compound of formula (I), the isotope-labeled compound thereof, the solvate thereof, the pharmaceutically acceptable salt thereof, the solvate of the pharmaceutically acceptable salt thereof, or the prodrug thereof according to claim 1, wherein the compound of formula (I) is selected from the following compounds: 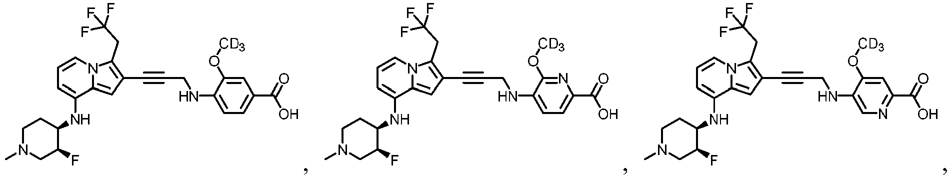 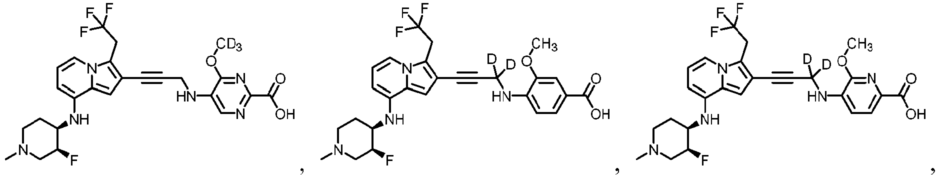 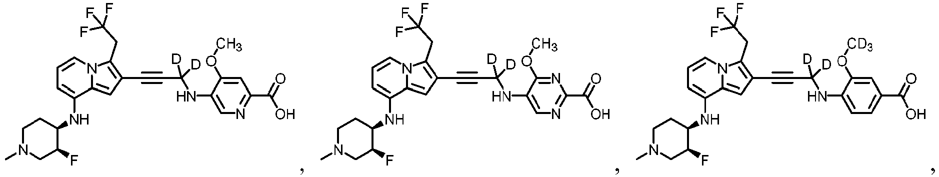 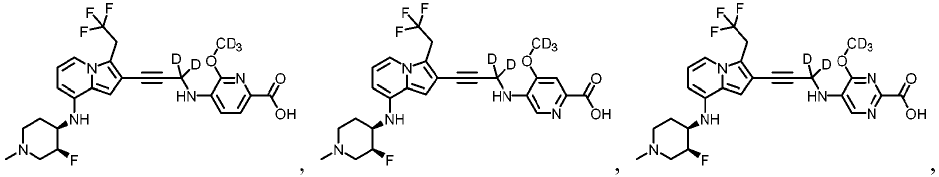 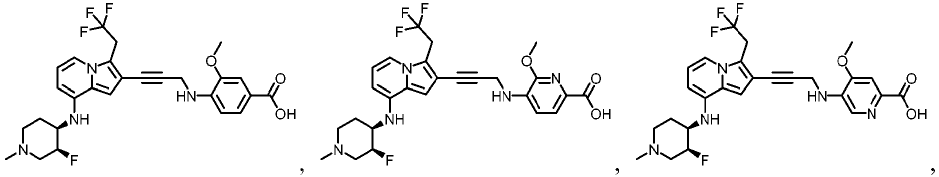 and

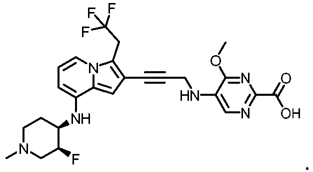

8. A pharmaceutical composition comprising the compound of formula (I) according to any one of claims 1 to 7, an isotope-labeled compound thereof, an enantiomer thereof, a diastereomer thereof, a solvate thereof, or a pharmaceutically acceptable salt thereof, and a pharmaceutical excipient.

9. Use of the compound of formula (I) according to any one of claims 1 to 7, an isotope-labeled compound thereof, an enantiomer thereof, a diastereomer thereof, a solvate thereof, or a pharmaceutically acceptable salt thereof, or the pharmaceutical composition according to claim 8 in the manufacture of a p53 mutant pocket binding agent or a medicament for the treatment and/or prevention of a disease related to p53 mutants.

10. The use according to claim 9, wherein the use satisfies one or more of the following conditions:
(1) The p53 mutant has a mutation at amino acid 220, such as p53 Y220C;
(2) The pocket binding agent promotes the ability of p53 mutants to bind to DNA;
(3) The disease related to p53 mutants is cancer, such as breast cancer, gastric cancer, lung cancer, or ovarian cancer.

11. Use of the compound of formula (I) according to any one of claims 1 to 7, an isotope-labeled compound thereof, an enantiomer thereof, a diastereomer thereof, a solvate thereof, or a pharmaceutically acceptable salt thereof, or the pharmaceutical composition according to claim 8 in the manufacture of a medicament for the treatment and/or prevention of cancer.

12. The use according to claim 11, wherein the cancer is breast cancer, gastric cancer, lung cancer, or ovarian cancer.

13. A method for the treatment and/or prevention of cancer, wherein the method comprises administering to a patient a therapeutically effective amount of the compound of formula (I) according to any one of claims 1 to 7, an isotope-labeled compound thereof, an enantiomer thereof, a diastereomer thereof, a solvate thereof, or a pharmaceutically acceptable salt thereof.
